# EUROPEAN PATENT APPLICATION

(11) **EP 3 960 835 A1**
(43) Date of publication of application: **02.03.2022**
(21) Application number: 20795178.1
(22) Date of filing: 17.04.2020
(51) Int. Cl.: C09K 11/06, C07D 209/86, C07D 487/04, H05B 33/10, H01L 51/50

(54) **ORGANIC ELECTROLUMINESCENT ELEMENT**

(30) Priority: 25.04.2019 JP 2019083835
(71) Applicant: NIPPON STEEL Chemical & Material Co., Ltd., Tokyo 103-0027 (JP)
(72) Inventor: OGAWA, Junya, Tokyo 103-0027 (JP); IKENAGA, Yuji, Tokyo 103-0027 (JP); YOSHIDA, Kazunari, Tokyo 103-0027 (JP); KITAHARA, Ikumi, Tokyo 103-0027 (JP)
(74) Representative: Beckmann, Claus
(86) International application number: PCT/JP2020/016870
(87) International publication number: WO 2020/218188

(57) **Abstract**

Provided is an organic EL device having a low drive voltage, high efficiency, and high drive stability, and a material for an organic electroluminescent device suitable for the organic EL device. This material for an organic EL device includes: an indolocarbazole compound represented by General Formula (1).

In addition, provided is an organic electroluminescent device in which this material for an organic electroluminescent device is used as a first host and carbazole compounds are used as second hosts. 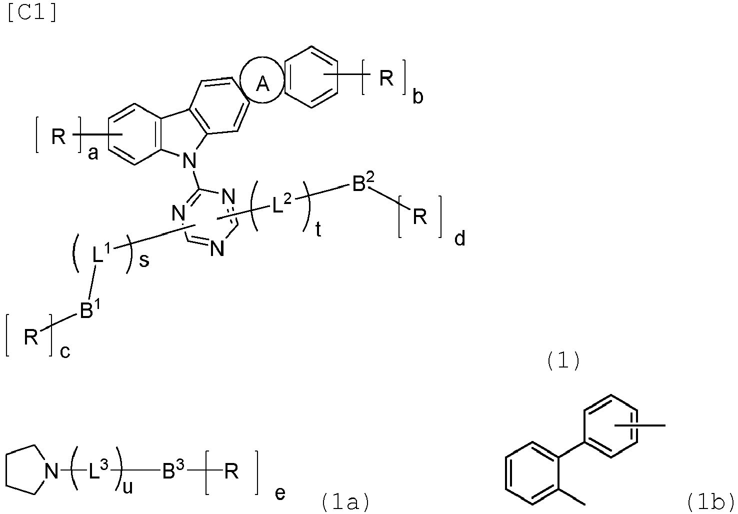 Here, a ring A is a heterocyclic ring represented by Formula (1a), L¹ to L³ are a direct bond, an aromatic hydrocarbon group or an aromatic heterocyclic group, B¹ to B³ represent a direct bond or a biphenyldiyl group represented by Formula (1b), and at least one of B¹ to B³ is a biphenyldiyl group.

## Description

### [Technical Field]

The present invention relates to an organic electroluminescent element (also referred to as an organic EL device). Specifically, the present invention relates to a material for an organic electroluminescent element composed of an indolocarbazole compound and an organic EL element using the material.

### [Background Art]

When a voltage is applied to an organic EL device, holes from an anode and electrons from a cathode are injected into a light emitting layer. Then, the injected holes and electrons are recombined in the light emitting layer to generate excitons. At this time, singlet excitons and triple excitons are generated at a ratio of 1:3 according to the statistical law of electron spin. It is said that the internal quantum efficiency of a fluorescent organic EL device in which light emission due to singlet excitons is used is limited to 25%. On the other hand, it is known that the internal quantum efficiency of a phosphorescent organic EL device in which light emission due to triple excitons is used can increase to up to 100% in a case where intersystem crossing from singlet excitons is efficiently performed.

However, extending the lifespan of a phosphorescent organic EL device has become a technical issue.

High-efficiency organic EL devices using delayed fluorescence have recently been developed. For example, PTL 1 discloses an organic EL device using a triplet-triplet fusion (TTF) mechanism which is one of delayed fluorescence mechanisms. In the TTF mechanism, a phenomenon that singlet excitons are generated due to collision of two triple excitons is used, and therefore it is theoretically thought that the internal quantum efficiency can be increased to 40%. However, since the efficiency thereof is lower than that of the phosphorescent organic EL device, further improvement in efficiency is required.

In PTL 2, an organic EL device in which a thermally activated delayed fluorescence (TADF) mechanism is used is disclosed. In the TADF mechanism, a phenomenon that reverse intersystem crossing from triple excitons to singlet exciton is caused in materials having a small energy difference between a singlet level and a triple level is used, and therefore, it is theoretically thought that the internal quantum efficiency can be increased to 100%. However, further improvement in lifespan characteristics is required similarly to the phosphorescent device.

### [Citation List]

### [Patent Literature]

[PTL 1] WO2010/134350A
[PTL 2] WO2011/070963A
[PTL 3] WO2008/056746A
[PTL 4] JP2003-133075A
[PTL 5] WO2013/062075A
[PTL 6] US2014/0374728A
[PTL 7] US2014/0197386A
[PTL 8] US2015/0001488A
[PTL 9] US2015/0236262A
[PTL 10] WO2016/194604A
[PTL 11] WO2011/136755A

Use of indolocarbazole compounds as a host material is disclosed in PTL 3. Use of biscarbazole compounds as a host material is disclosed in PTL 4.

Use of biscarbazole compounds as a host mixture is disclosed in PTL 5 and 6. Use of indolocarbazole compounds and biscarbazole compounds as a host mixture is disclosed in PTL 7, 8, 9, and 10.

Use of a host material obtained by preliminarily mixing a plurality of hosts containing indolocarbazole compounds is disclosed in PTL 11.

However, none of them can be said to be sufficient, and further improvement is desired.

### [Summary of Invention]

In order to apply organic EL devices to light sources or display devices such as flat panel displays, it is necessary to improve the luminous efficiency of devices and at the same time to secure sufficient stability during driving.
An object of the present invention is to provide an organic EL device having a low drive voltage, high efficiency, and high drive stability, and a material for an organic electroluminescent device suitable for the organic EL device.

The present inventors have conducted extensive studies, and as a result, they have found that an organic EL device in which a specific indolocarbazole compound is used exhibits excellent characteristics, thus leading to realization of the present invention.

The present invention is a material for an organic electroluminescent device including: an indolocarbazole compound represented by General Formula (1).

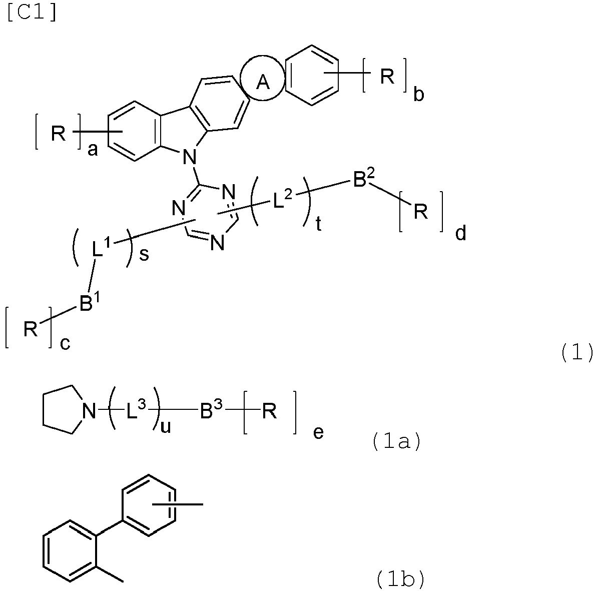

Here, a ring A is a heterocyclic ring represented by Formula (1a) and condensed with an adjacent ring at an arbitrary position, R's are independently hydrogen, an aliphatic hydrocarbon group having 1 to 10 carbon atoms, an aromatic hydrocarbon group having 6 to 10 carbon atoms, or an aromatic heterocyclic group having 3 to 12 carbon atoms, L¹ to L³ are independently a direct bond, an aromatic hydrocarbon group having 6 to 10 carbon atoms, or an aromatic heterocyclic group having 3 to 12 carbon atoms, B¹ to B³ independently represent a direct bond or a biphenyldiyl group represented by Formula (1b), and at least one of B¹ to B³ is a biphenyldiyl group represented by Formula (1b). a, b, c, d, and e each independently represent an integer of 0 to 3, and s, t, and u each independently represent an integer of 1 and 2.

A preferred aspect of General Formula (1) is that B³ is a biphenyldiyl group represented by Formula (1b) or a, b, and c are 0.

In addition, the present invention is an organic electroluminescent device obtained such that an anode, an organic layer, and a cathode are laminated on a substrate, characterized in that at least one layer in the organic layer is an organic layer containing the above-described material for an organic electroluminescent device.

The organic layer containing the above-described material for an organic electroluminescent device is at least one layer selected from the group consisting of a light emitting layer, an electron transport layer, and a hole-blocking layer.

Furthermore, the present invention is the organic electroluminescent device according to claim 4, characterized in that the organic layer containing the material for an organic electroluminescent device is the light emitting layer comprises of a vapor deposition layer containing a first host, a second host, and the luminescent dopant material, the first host is selected from the compounds represented by General Formula (1), and the second host is selected from compounds represented by General Formula (2), General Formula (3), and General Formula (4) below.

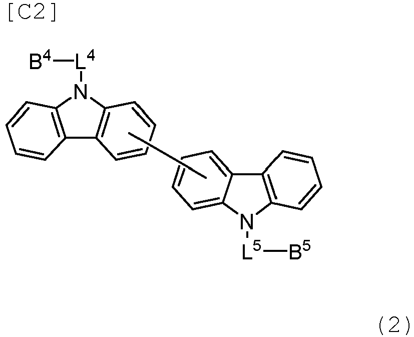

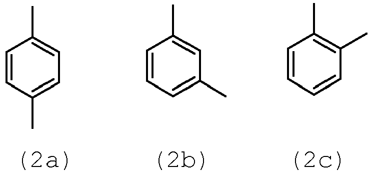

Here, B⁴ and B⁵ independently represent hydrogen, an aromatic hydrocarbon group having 6 to 14 carbon atoms, or a group in which two aromatic hydrocarbon groups which may be the same as or different from each other are linked. L⁴ and L⁵ independently represent a phenylene group represented by Formulae (2a) to (2c). 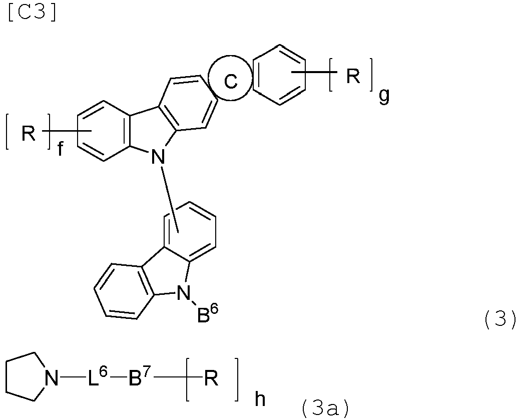 Here, a ring C is a heterocyclic ring represented by Formula (3a) and condensed with an adjacent ring at an arbitrary position, R's are independently hydrogen, an aliphatic hydrocarbon group having 1 to 10 carbon atoms, an aromatic hydrocarbon group having 6 to 10 carbon atoms, or an aromatic heterocyclic group having 3 to 12 carbon atoms, L⁶ is a direct bond, an aromatic hydrocarbon group having 6 to 10 carbon atoms, or an aromatic heterocyclic group having 3 to 12 carbon atoms, B⁶ is hydrogen, an aromatic hydrocarbon group having 6 to 10 carbon atoms, or an aromatic heterocyclic group having 3 to 12 carbon atoms, and B⁷ is an aromatic hydrocarbon group having 6 to 10 carbon atoms or an aromatic heterocyclic group having 3 to 12 carbon atoms. f, g, and h each independently represent an integer of 0 to 3.

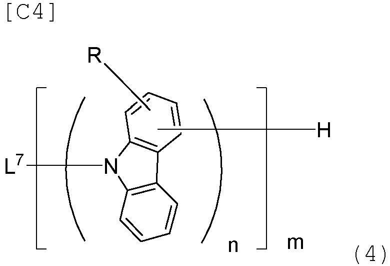

Here, L⁷ is an m-valent aromatic hydrocarbon group having 6 to 30 carbon atoms, an aromatic heterocyclic group having 3 to 16 carbon atoms, or a linked aromatic group which is obtained by linking 2 to 10 aromatic rings thereof and contains no carbazole ring. R's are independently hydrogen, an alkyl group having 1 to 10 carbon atoms, or a cycloalkyl group having 3 to 11 carbon atoms. m is a number of substitutions and is an integer of 1 to 3. n's are numbers of repetitions and each independently an integer of 1 to 4, and at least one n is an integer of 2 to 4.

Preferred aspects of General Formula (1), (2), (3), and (4) above will be shown below.

At least one bonding structure represented by Formula (c1) is included in General Formula (4). 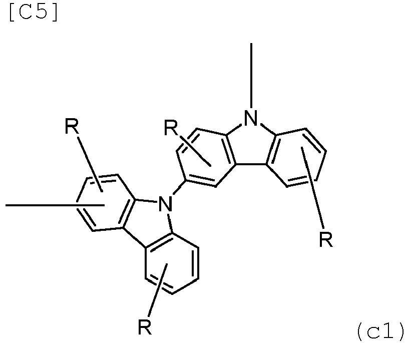 (Here, R has the same meaning as that in General Formula (4).)

General Formula (2) is Formula (5) below. 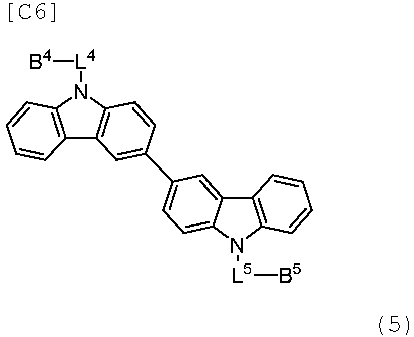 (Here, B⁴, B⁵, L⁴, and L⁵ have the same meaning as those in General Formula (2).)

General Formula (3) is Formula (6) or (7) below. 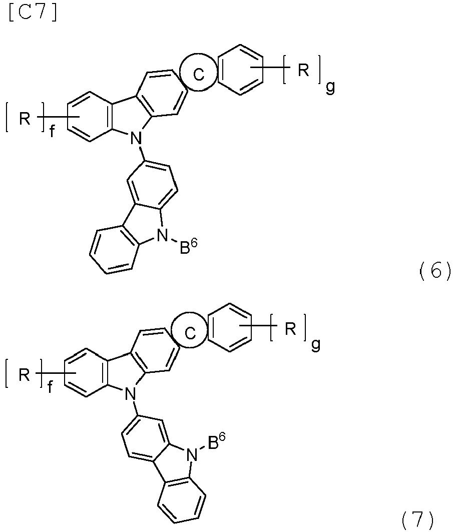 (Here, a ring C, R, B⁶, f, and g have the same meaning as those in General Formula (3).)

At least one bonding structure represented by Formula (c2) is included in General Formula (4). 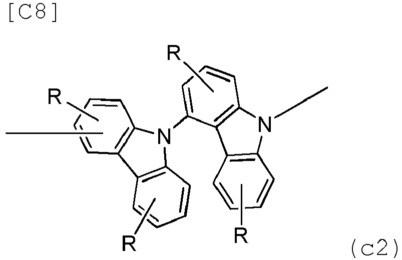 (Here, R has the same meaning as that in General Formula (4).)

General Formula (1) is any of Formulae (8) to (11) below. 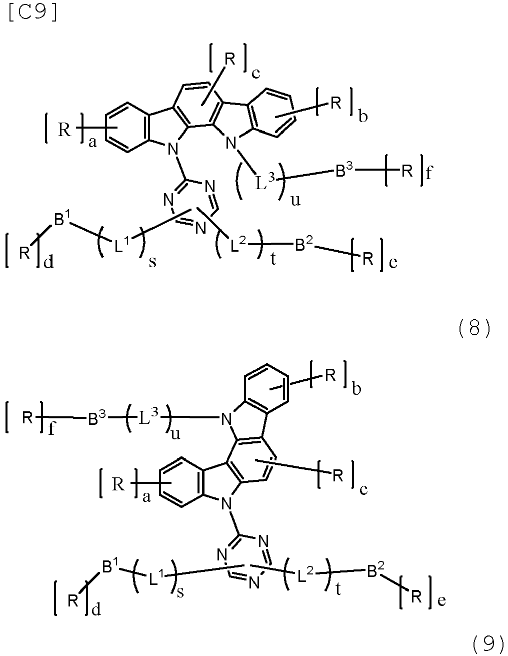 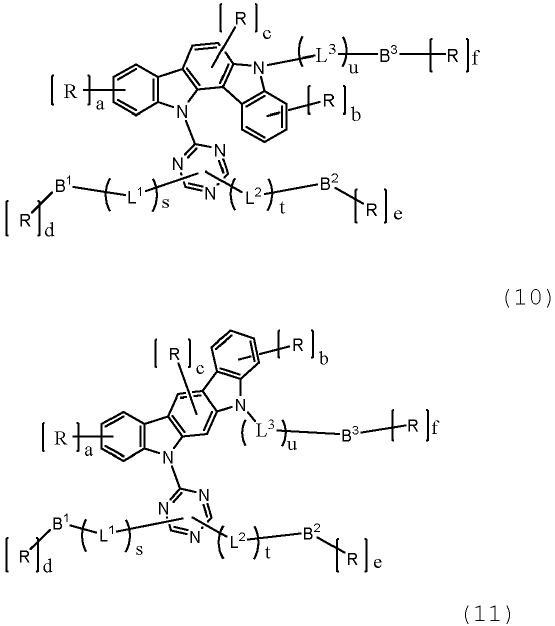 (Here, B¹ to B³, L¹ to L³, R, a to f, and s to u have the same meaning as those in General Formula (1).)

Preferred aspects of the above-described organic electroluminescent device will be shown below.

The proportion of the first host is greater than 20 wt% and less than 55 wt% based on the total amount of the first host and the second host.

The luminescent dopant material is an organic metal complex containing at least one metal selected from the group consisting of ruthenium, rhodium, palladium, silver, rhenium, osmium, iridium, platinum, and gold, or a thermally activated delayed fluorescent dopant material.

The light emitting layer and the hole-blocking layer adjacent thereto are provided, and the compound represented by General Formula (1) is contained in the hole-blocking layer.

In addition, the present invention is a method for producing an organic electroluminescent device, the method including: a step of mixing a first host with a second host to prepare a premixture and then vapor-depositing the host material containing the hosts to form a light emitting layer when producing the above-described organic electroluminescent device.

In the above-described method for producing an organic electroluminescent device, a difference in 50% weight reduction temperature between the first host and the second host is suitably within 20°C.

In order to improve the characteristics of the organic EL device, it is necessary that durability of materials used in an organic layer with respect to charges be high. In a light emitting layer, it is particularly important to suppress leakage of excitons and charges to neighbor layers. In order to suppress this leakage of charges and excitons, it is effective to delocalize of a light emitting region in the light emitting layer. To do so, it is necessary to control the amount of both charges (electrons and holes) injected into the light emitting layer or the amount of both charges transported in the light emitting layer such that it is within a preferred range.

The injection and transport capability of materials for both charges used in an organic layer greatly depends on energy levels of molecular orbitals of materials and the extent of intermolecular interactions. The material for an organic EL device of the present invention has an ortho-linked biphenyldiyl group represented by Formula (1b). Although indolocarbazole compounds have a particularly high electron injection and transport capability, close proximity of the indolocarbazole molecules can be inhibited due to steric hindrance effects of biphenyldiyl groups.

Moreover, it is thought that by changing bonding sites or types of substituents of biphenyldiyl groups, the material for an organic EL device of the present invention allows a high level of control of intermolecular interactions of molecular orbitals which greatly contribute to electron injection and transport with respect to the light emitting layer, whereby an excellent organic EL device can be provided.

In a case where the material for an organic EL device of the present invention is used in a host of a light emitting layer, carbazole compounds represented by General Formulae (2) to (4) can be used as a second host to provide a superior organic EL device. It is thought that this is because the carbazole compounds represented by General Formulae (2) to (4) have a particularly high hole injection and transport capability, therefore allowing a high level of control of hole injection and transport properties by changing a bonding mode of a carbazole ring or the number and types of substituents on this skeleton. By mixing the material for an organic EL device of the present invention with the above-described carbazole compounds for use, the amount of both charges injected into an organic layer can be adjusted to within a preferred range, and better device characteristics can be expected. In particular, since a delayed fluorescent EL device or a phosphorescent EL device has a sufficiently high lowest excited triplet energy to confine an excitation energy generated in a light emitting layer, there is no leakage of energy from the light emitting layer, and a low voltage, a high efficiency, and long lifespan can be achieved.

### [Brief Description of Drawings]

[Fig. 1]

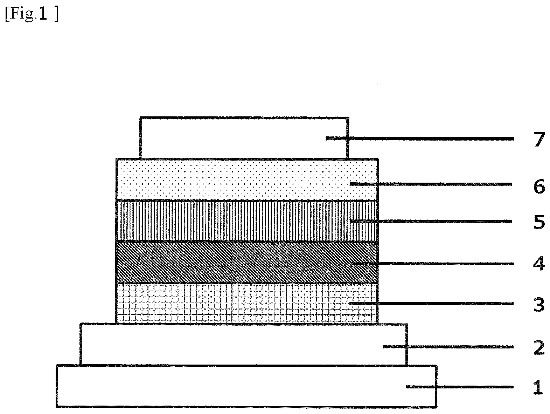

Fig. 1 is a schematic cross-sectional view illustrating an example of an organic EL device.

### [Description of Embodiment]

A material for an organic EL device of the present invention includes an indolocarbazole compound represented by General Formula (1) above.

An organic EL device of the present invention has a structure in which an anode, an organic layer, and a cathode are laminated on a substrate, and contains the above-described material for an organic electroluminescent device in at least one layer in the organic layer.

The organic EL device has an organic layer including a plurality of layers between an anode and a cathode facing each other. At least one of the plurality of layers may be a light emitting layer, and there are may be a plurality of light emitting layers.

General Formula (1) above will be described.

A ring A is a heterocyclic ring represented by Formula (1a) and condensed with an adjacent ring at an arbitrary position.

R's independently represent hydrogen, an aliphatic hydrocarbon group having 1 to 10 carbon atoms, an aromatic hydrocarbon group having 6 to 10 carbon atoms, or an aromatic heterocyclic group having 3 to 12 carbon atoms. An aliphatic hydrocarbon group having 1 to 8 carbon atoms, a phenyl group, or an aromatic heterocyclic group having 3 to 9 carbon atoms is preferable. An aliphatic hydrocarbon group having 1 to 6 carbon atoms, a phenyl group, or an aromatic heterocyclic group having 3 to 6 carbon atoms is more preferable.

Specific examples of the above-described aliphatic hydrocarbon group having 1 to 10 carbon atoms include methyl, ethyl, propyl, butyl, pentyl, hexyl, heptyl, octyl, nonyl, and decyl groups. An alkyl group having 1 to 4 carbon atoms is preferable.

Specific examples of the above-described aromatic hydrocarbon group having 6 to 10 carbon atoms or aromatic heterocyclic group having 3 to 12 carbon atoms include aromatic groups formed by removing one H from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, or carbazole. Preferred examples thereof include aromatic groups formed from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, or benzothiadiazole. More preferred examples thereof include aromatic groups formed from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, or oxadiazole.

L¹, L², and L³ are independently a direct bond, an aromatic hydrocarbon group having 6 to 10 carbon atoms, or an aromatic heterocyclic group having 3 to 12 carbon atoms. Preferred examples of aromatic hydrocarbon groups or aromatic heterocyclic groups are the same as those in the case where R's are these groups except that these groups are divalent groups.

B¹, B², and B³ independently represent a direct bond or a group represented by Formula (1b), and at least one of B¹ to B³ is a group (biphenyldiyl group) represented by Formula (1b). B³ is preferably the group represented by Formula (1b).

a, b, c, d, and e represent numbers of substitutions and each independently represent an integer of 0 to 3, and an integer of 0 or 1 is preferable. a, b, and c are preferably 0.

s, t, u represent numbers of repetitions and each independently represent an integer of 1 and 2, and 1 is preferable.

In the present specification, in a case where the number of repetitions is 2 or more, repeating units thereof may be the same as or different from each other.

Preferred aspects of compounds represented by General Formula (1) are compounds represented by any of Formulae (8) to (11) above. In Formulae (8) to (11), symbols shared by those in General Formula (1) have the same meaning.

Specific examples of compounds represented by General Formula (1) will be shown below, but the present invention is not limited to these exemplified compounds.

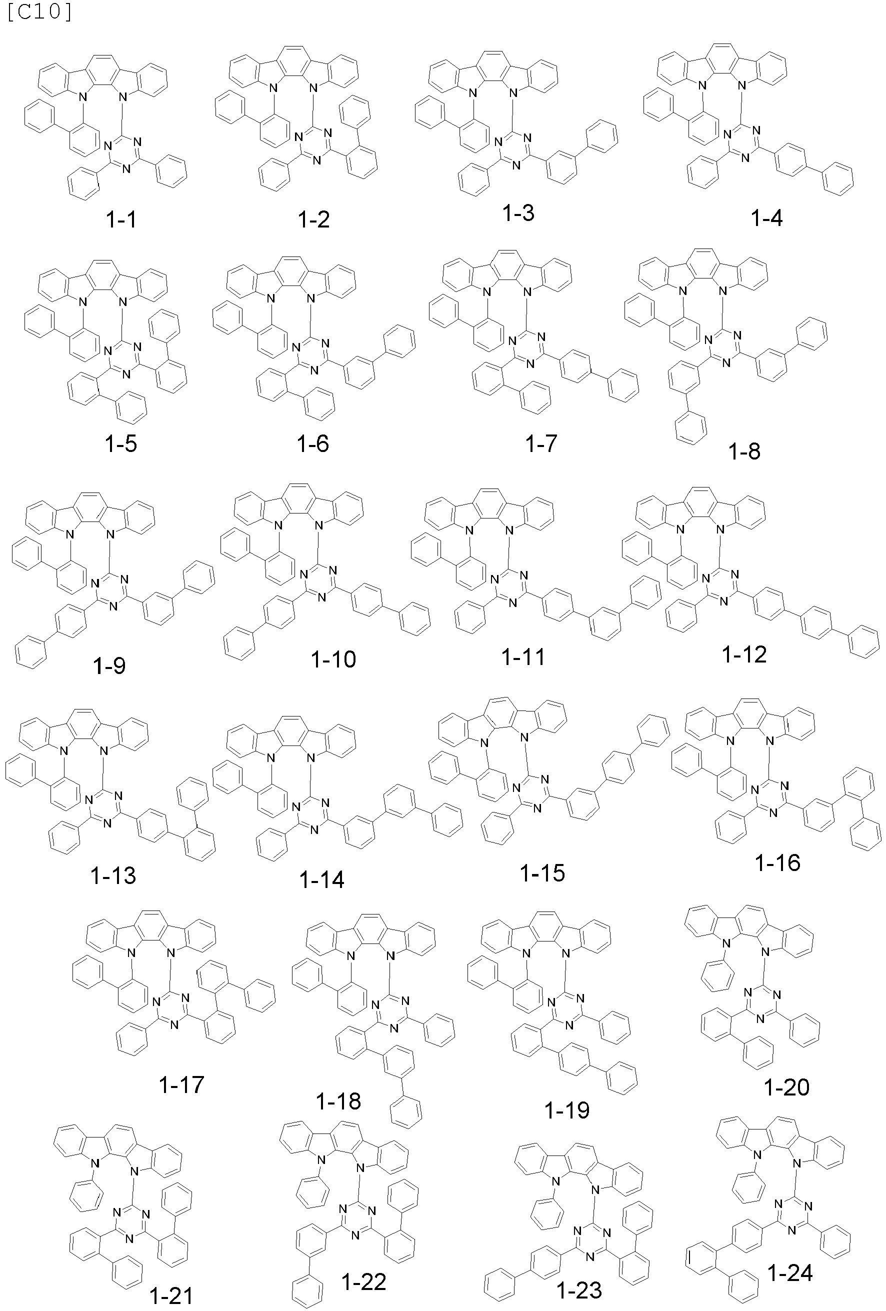

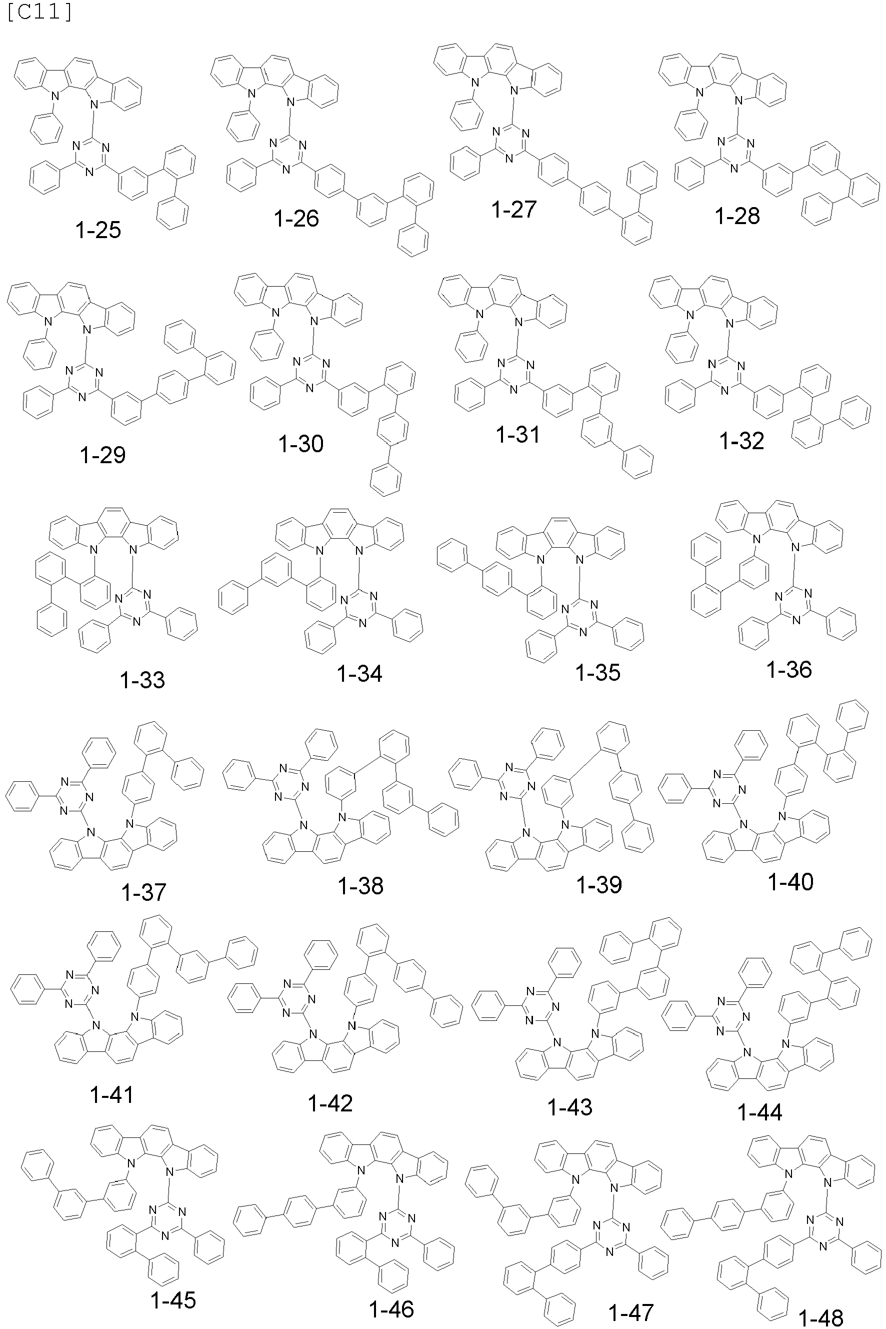

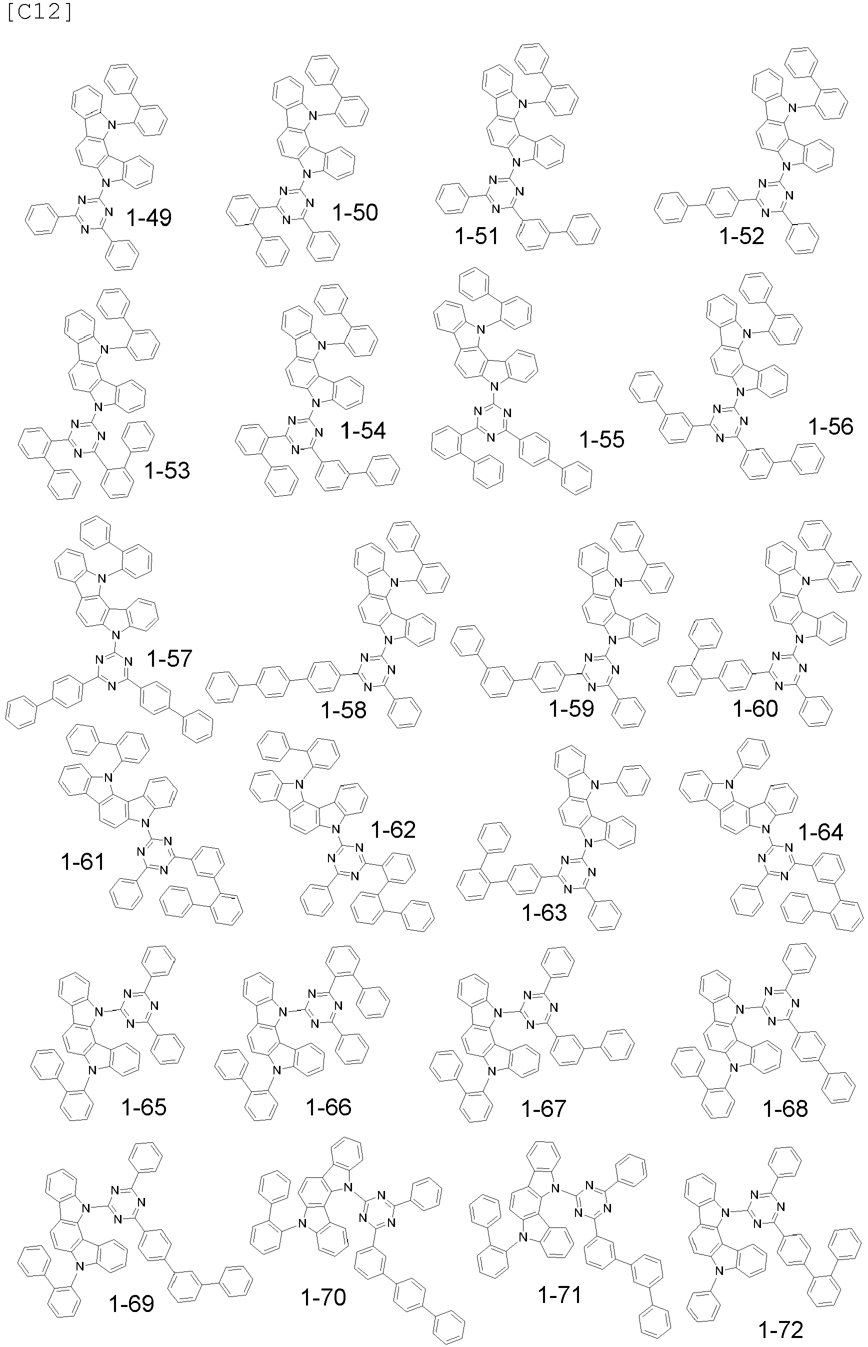

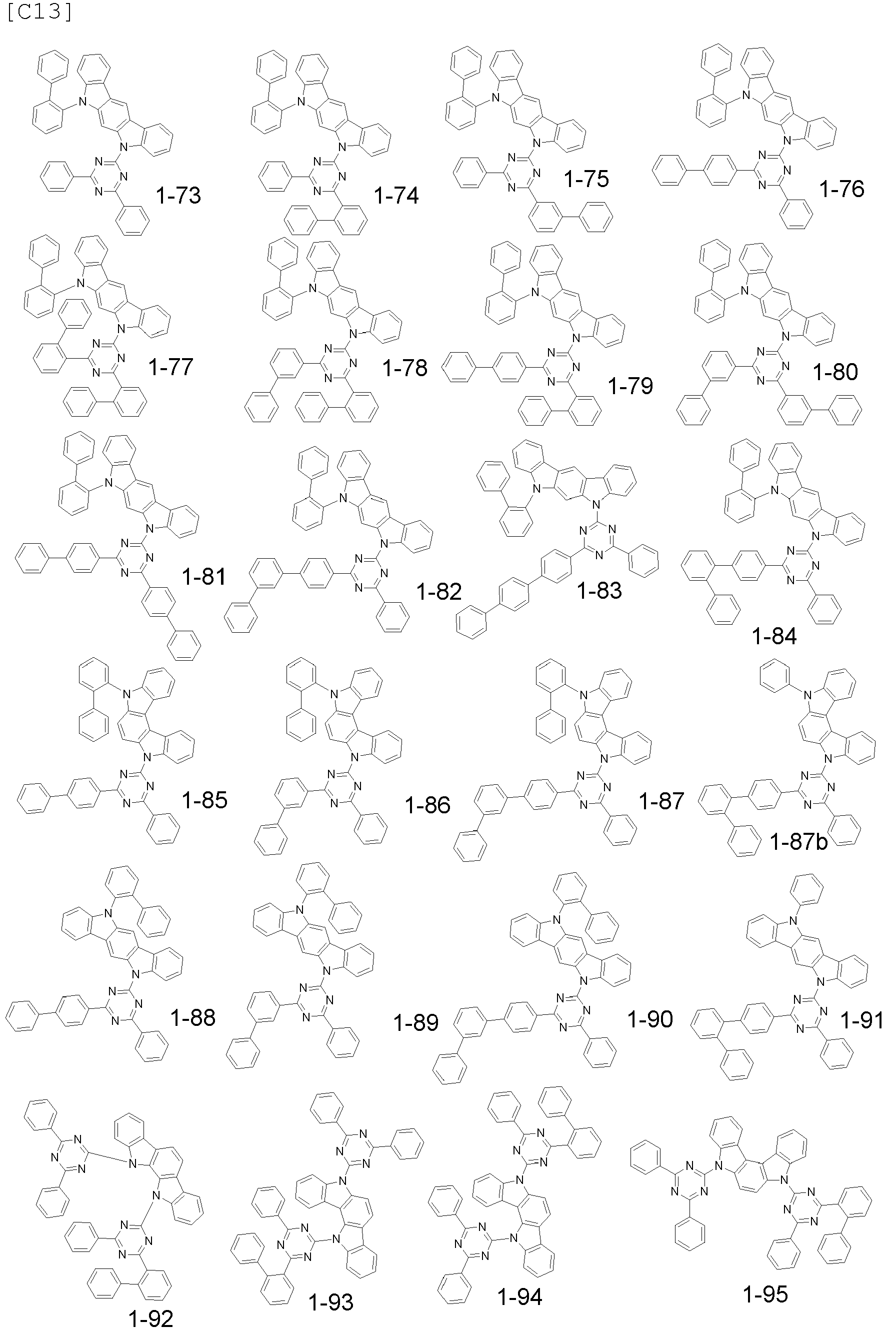

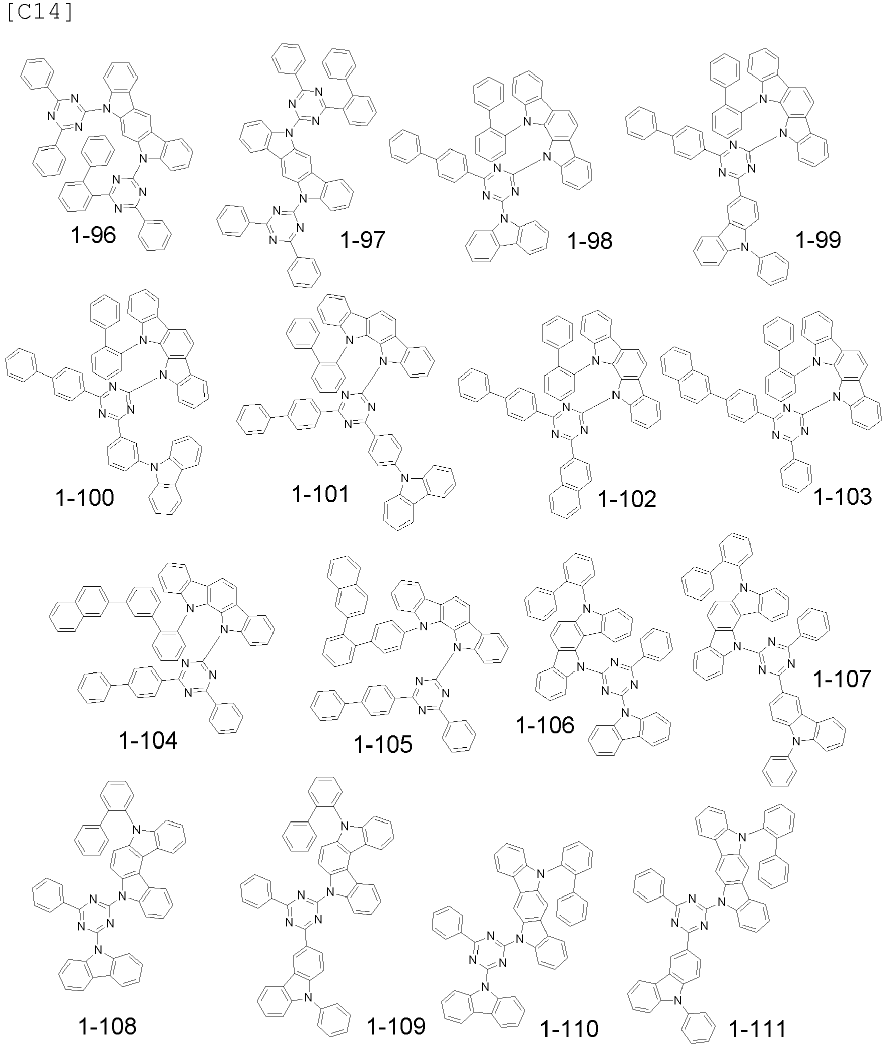

The organic electroluminescent device of the present invention contains the above-described material for an organic electroluminescent device in at least one layer in the organic layer. The organic electroluminescent device of the present invention preferably contains the above-described material for an organic electroluminescent device in at least one selected from the group consisting of a light emitting layer, an electron transport layer, and a hole-blocking layer. The organic electroluminescent device of the present invention still more preferably contains the above-described material for an organic electroluminescent device in a light emitting layer.

In a case where a light emitting layer contains the material for an organic electroluminescent device of the present invention, the material for an organic electroluminescent device of the present invention is suitable as a host.

In a case where the material for an organic electroluminescent device of the present invention is used as a host, it is preferably used as a first host and another compound is preferably used as a second host. A more preferable aspect is as follows.

The first host is selected from the compounds represented by General Formula (1) which are materials for an organic electroluminescent device of the present invention.

The second host is selected from the compounds represented by General Formulae (2), (3), or (4). Since these compounds have a carbazole ring, they are called carbazole compounds.

General Formula (2) as the second host and Formula (5) as a preferred aspect thereof will be described. In General Formulae (2) and Formula (5), common symbols have the same meaning.

B⁴ and B⁵ independently represent hydrogen, an aromatic hydrocarbon group having 6 to 14 carbon atoms, or a group (linked aromatic group) in which two aromatic rings of aromatic hydrocarbon groups are linked. It is preferable that B⁴ and B⁵ be independently hydrogen or an aromatic hydrocarbon group having 6 to 12 carbon atoms, and it is more preferable that B⁴ and B⁵ be an aromatic hydrocarbon group having 6 to 10 carbon atoms. A preferred aspect is that B⁴ is hydrogen or that B⁴ is hydrogen and B⁵ is the above-described aromatic hydrocarbon group or linked aromatic group.

Specific examples of a case where B⁴ and B⁵ are aromatic hydrocarbon groups or linked aromatic groups include aromatic hydrocarbon groups and linked aromatic groups formed by removing one H from such as benzene, naphthalene, anthracene, phenanthrene, fluorene, and biphenyl, or linked aromatic groups in which two aromatic rings of the aromatic hydrocarbon groups are linked. Preferred examples thereof include aromatic groups formed from benzene, naphthalene, anthracene, or phenanthrene or linked aromatic groups in which two aromatic groups thereof are linked, and aromatic groups formed from benzene, naphthalene, phenanthrene, or biphenyl are more preferable. It is more preferable that B⁴ or B⁵ be a phenyl group.

B⁴ or B⁵ may be hydrogen. However, in this case, the other one is preferably the above-described aromatic group or linked aromatic group. It is still more preferable that B⁴ be hydrogen and B⁵ be a phenyl group. In addition, the above-described aromatic groups or the linked aromatic groups may have a substituent, and a preferred substituent is an alkyl group having 1 to 12 carbon atoms or an alkoxy group having 1 to 12 carbon atoms.

In the present specification, it is understood that an aliphatic hydrocarbon group, an aromatic hydrocarbon group, an aromatic heterocyclic group, a linked aromatic group, and the like may have a group having a substituent unless otherwise specified as being unsubstituted. In addition, it is understood that one corresponding to a linked aromatic group does not correspond to a substituted aromatic hydrocarbon group or a substituted aromatic heterocyclic group.

L⁴ and L⁵ are phenylene groups which are represented by Formula (2a), (2b), or (2c). A p-phenylene group or a m-phenylene group represented by Formula (2a) or (2b) is preferable. Moreover, it is preferable that L⁴ and L⁵ be different from each other. In this case, in a case where B⁴ or B⁵ is hydrogen, this is treated as a phenyl group and different from a phenylene group.

Specific examples of carbazole compounds represented by General Formula (2) and Formula (5) will be shown below, but the present invention is not limited to these exemplified compounds.

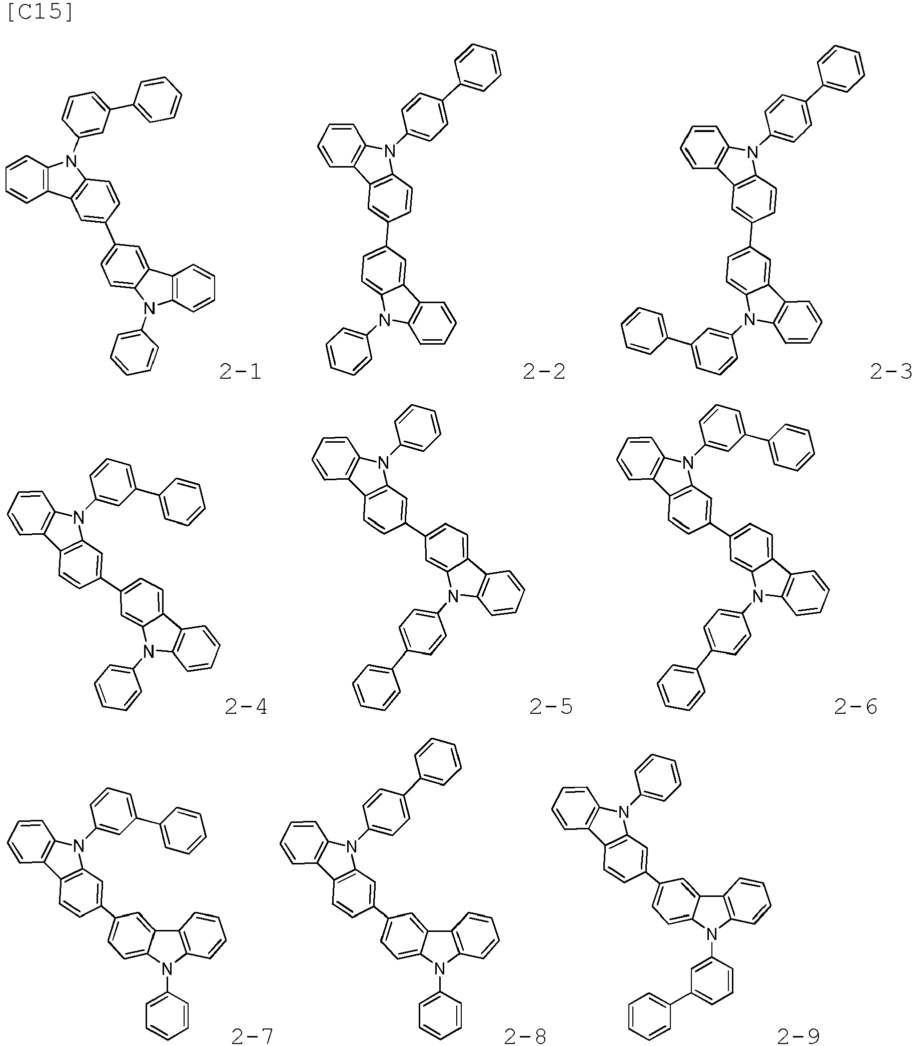

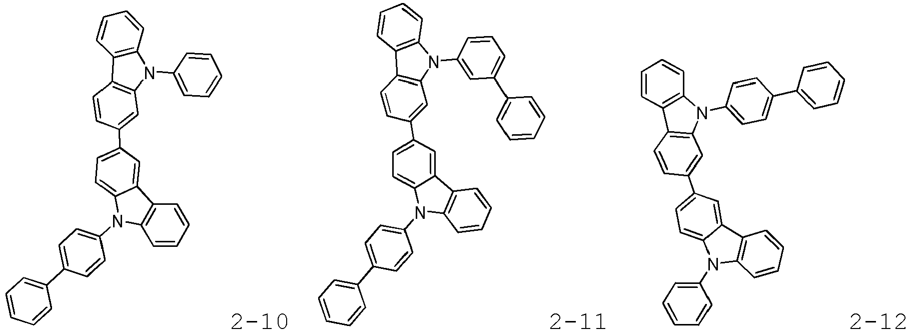

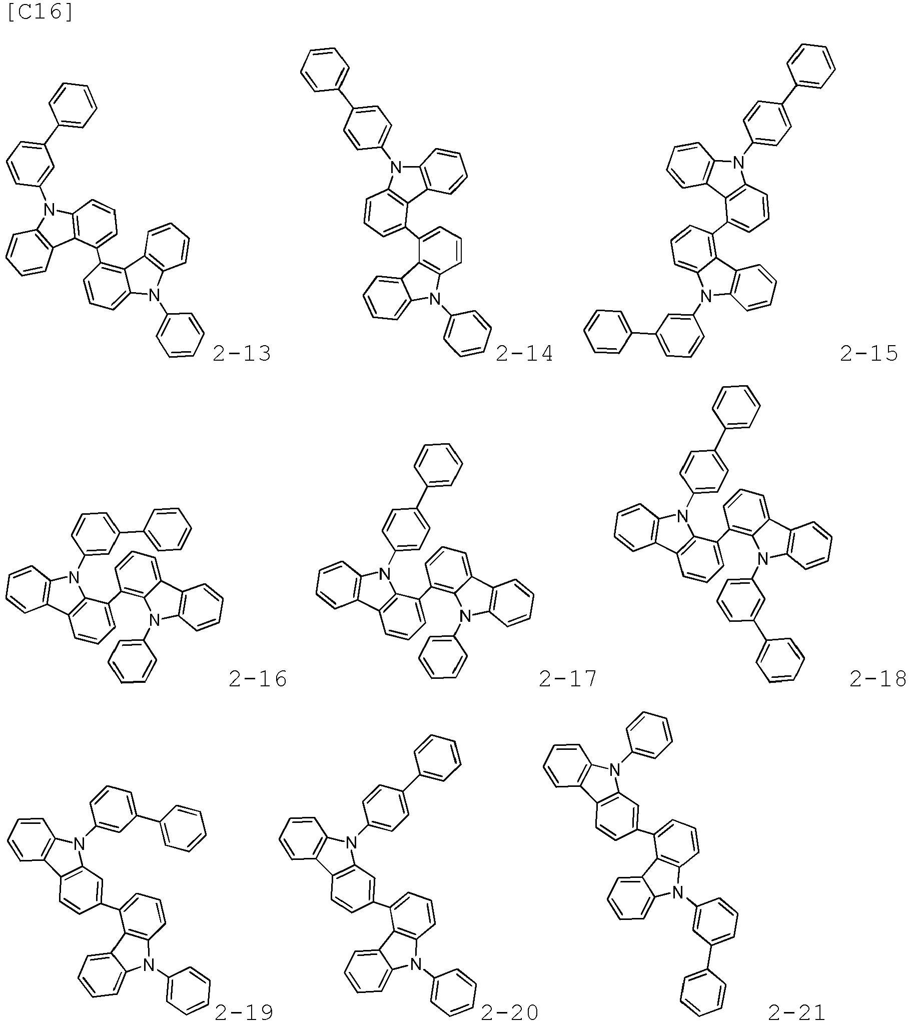

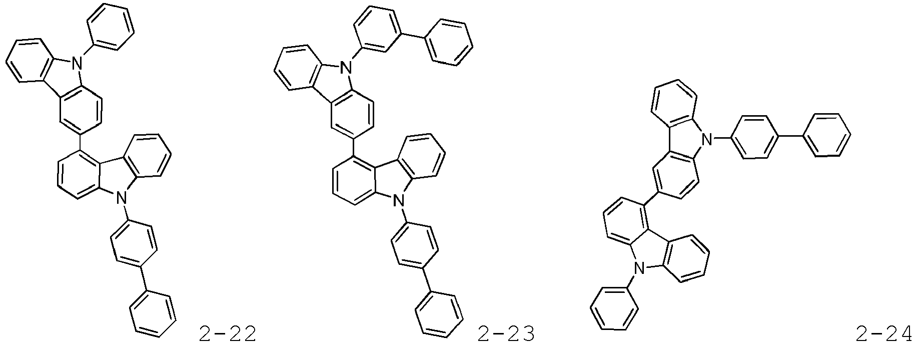

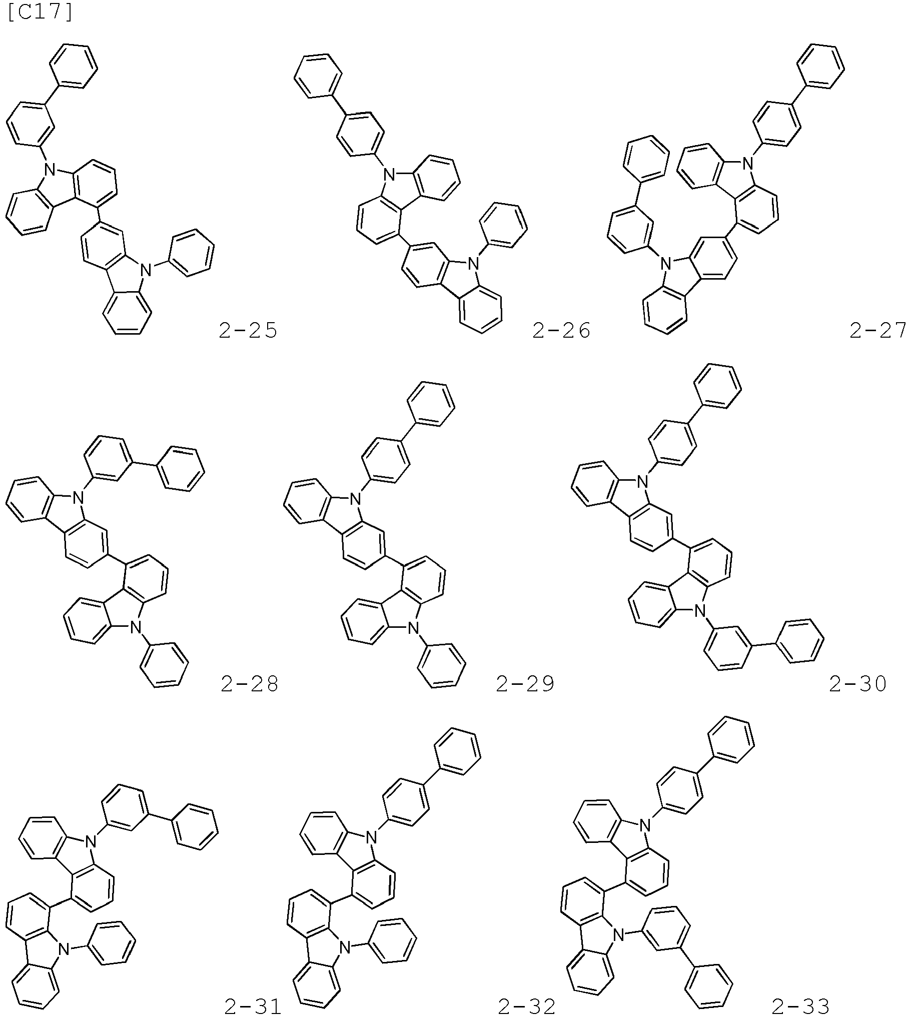

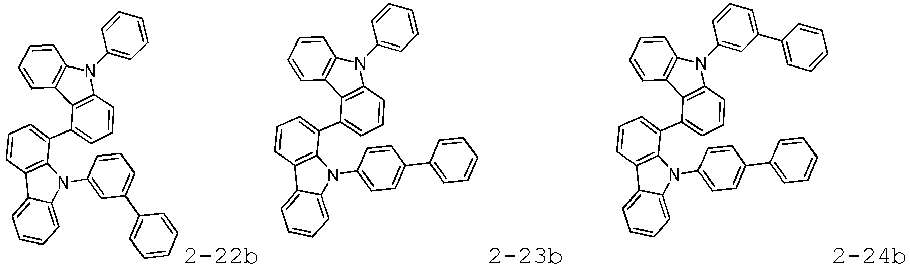

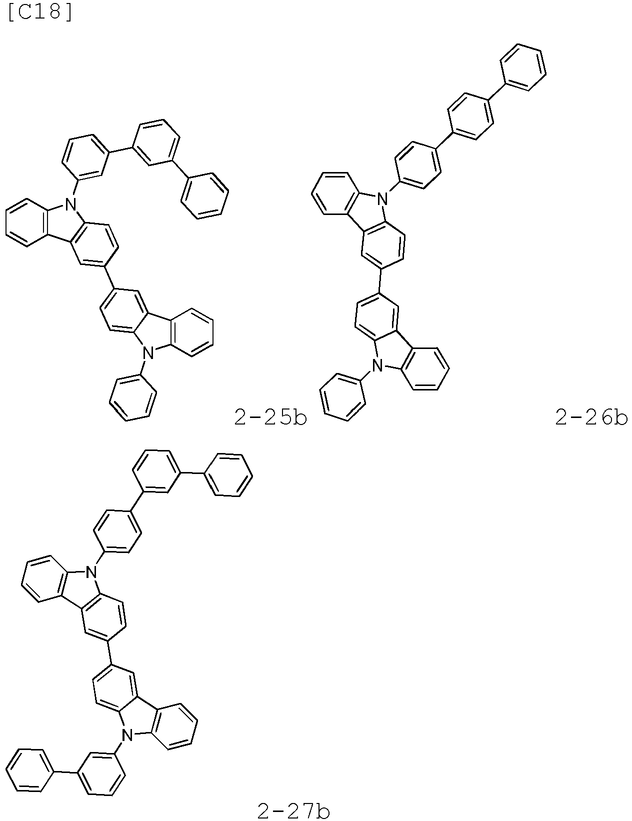

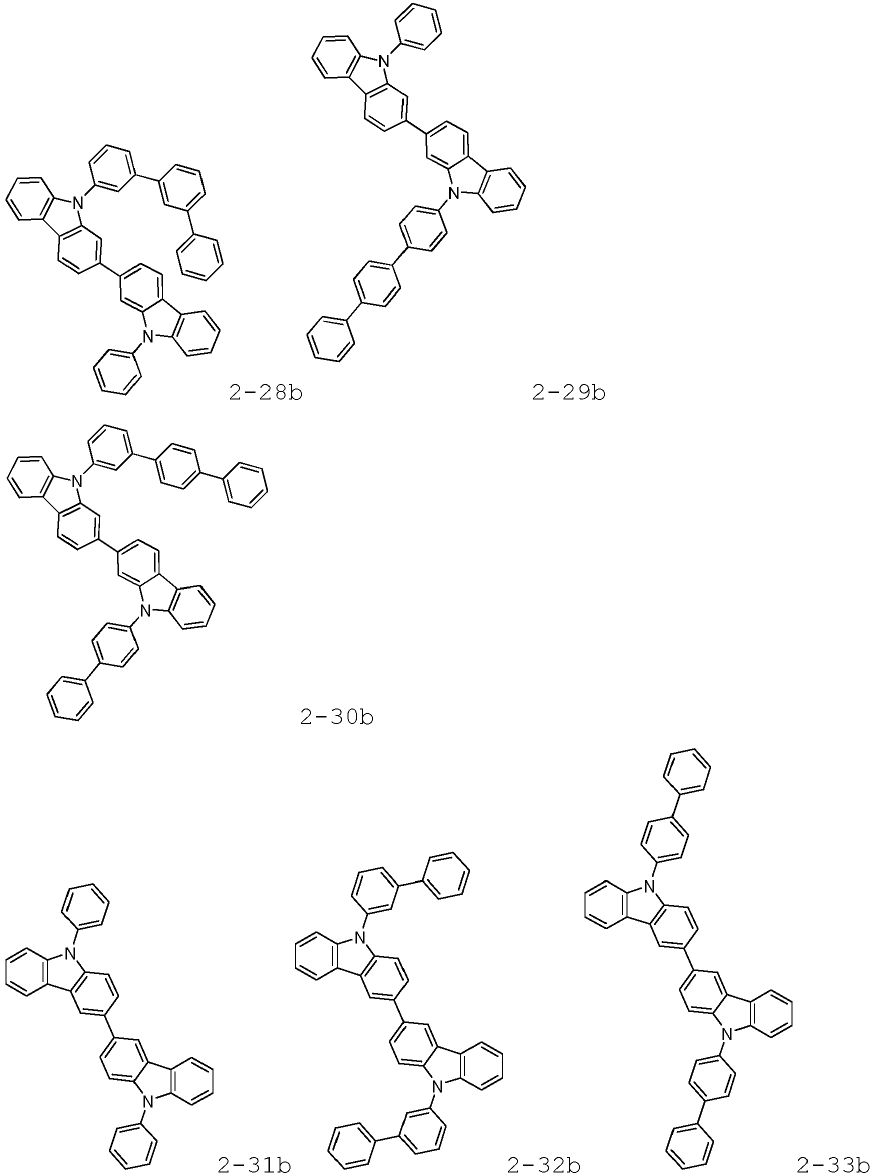

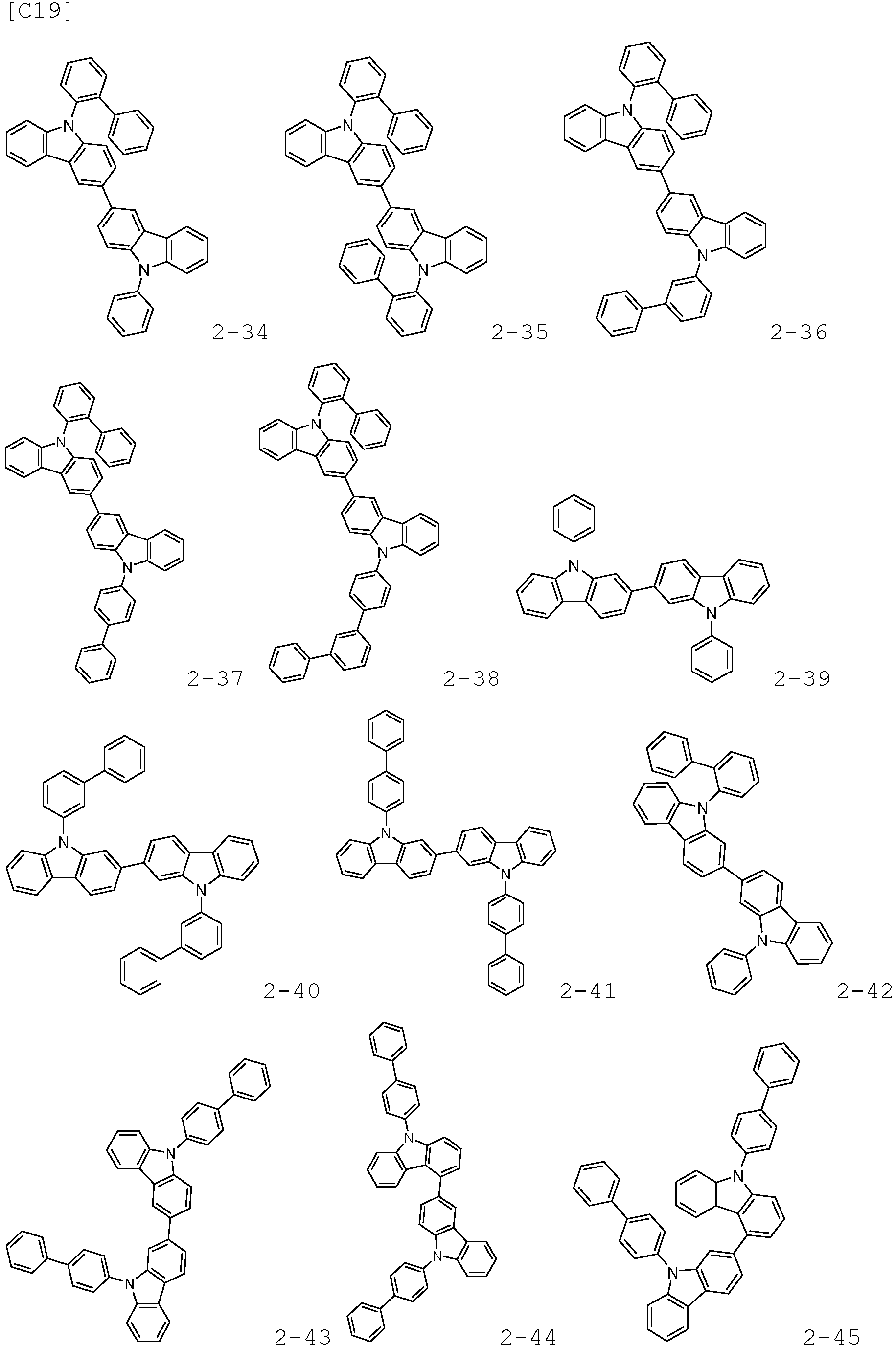

Next, General Formula (3) above will be described.

In General Formula (3), a ring C is a heterocyclic ring represented by Formula (3a) and condensed with an adjacent ring at an arbitrary position.

R's are independently hydrogen, an aliphatic hydrocarbon group having 1 to 10 carbon atoms, an aromatic hydrocarbon group having 6 to 10 carbon atoms, or an aromatic heterocyclic group having 3 to 12 carbon atoms. Regarding such an aliphatic hydrocarbon group, an aromatic hydrocarbon group, or an aromatic heterocyclic group, the same applies as in the case where R's in General Formula (1) are these groups, and preferred ranges are the same.

L⁶ is independently a direct bond, an aromatic hydrocarbon group having 6 to 10 carbon atoms, or an aromatic heterocyclic group having 3 to 12 carbon atoms. Regarding such an aromatic hydrocarbon group or an aromatic heterocyclic group, the same applies as in the case where L¹ to L³ in General Formula (1) are these groups, and preferred ranges are the same.

B⁶ is hydrogen, an aromatic hydrocarbon group having 6 to 10 carbon atoms, or an aromatic heterocyclic group having 3 to 12 carbon atoms, and B⁷ is an aromatic hydrocarbon group having 6 to 10 carbon atoms or an aromatic heterocyclic group having 3 to 12 carbon atoms. Regarding the aromatic hydrocarbon group or the aromatic heterocyclic group, the same applies as in the case where B¹ to B³ in General Formula (1) are these groups, and preferred ranges are the same.

f, g, and h each independently represent an integer of 0 to 3.

However, General Formula (3) is not the same as General Formula (1).

A compound represented by General Formula (3) is preferably a compound represented by Formula (6) or (7) above.

In Formula (6) or (7), a ring B, R, Ar⁷, f, and g have the same meaning as those in General Formula (3).

Specific examples of carbazole compounds represented by General Formula (3) will be shown below, but the present invention is not limited thereto.

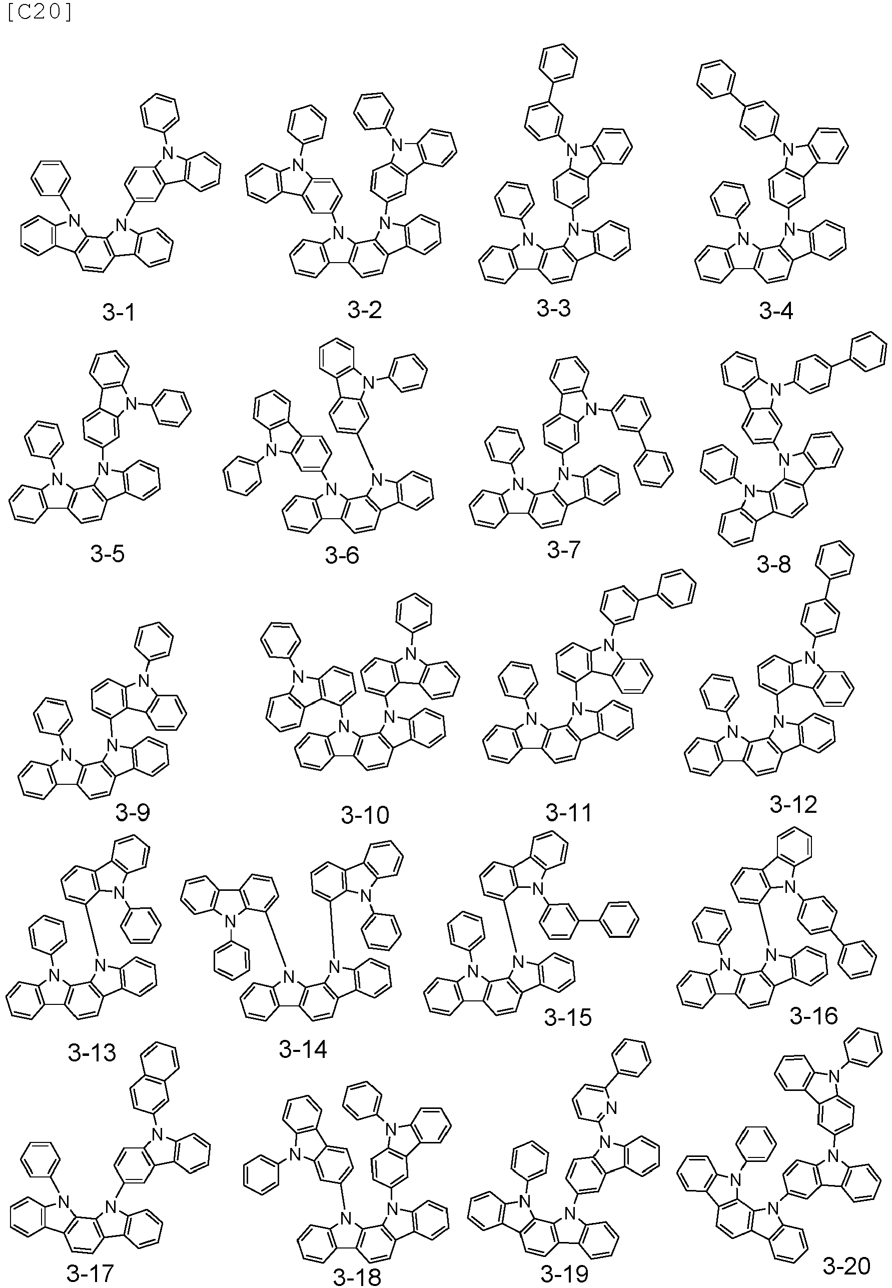

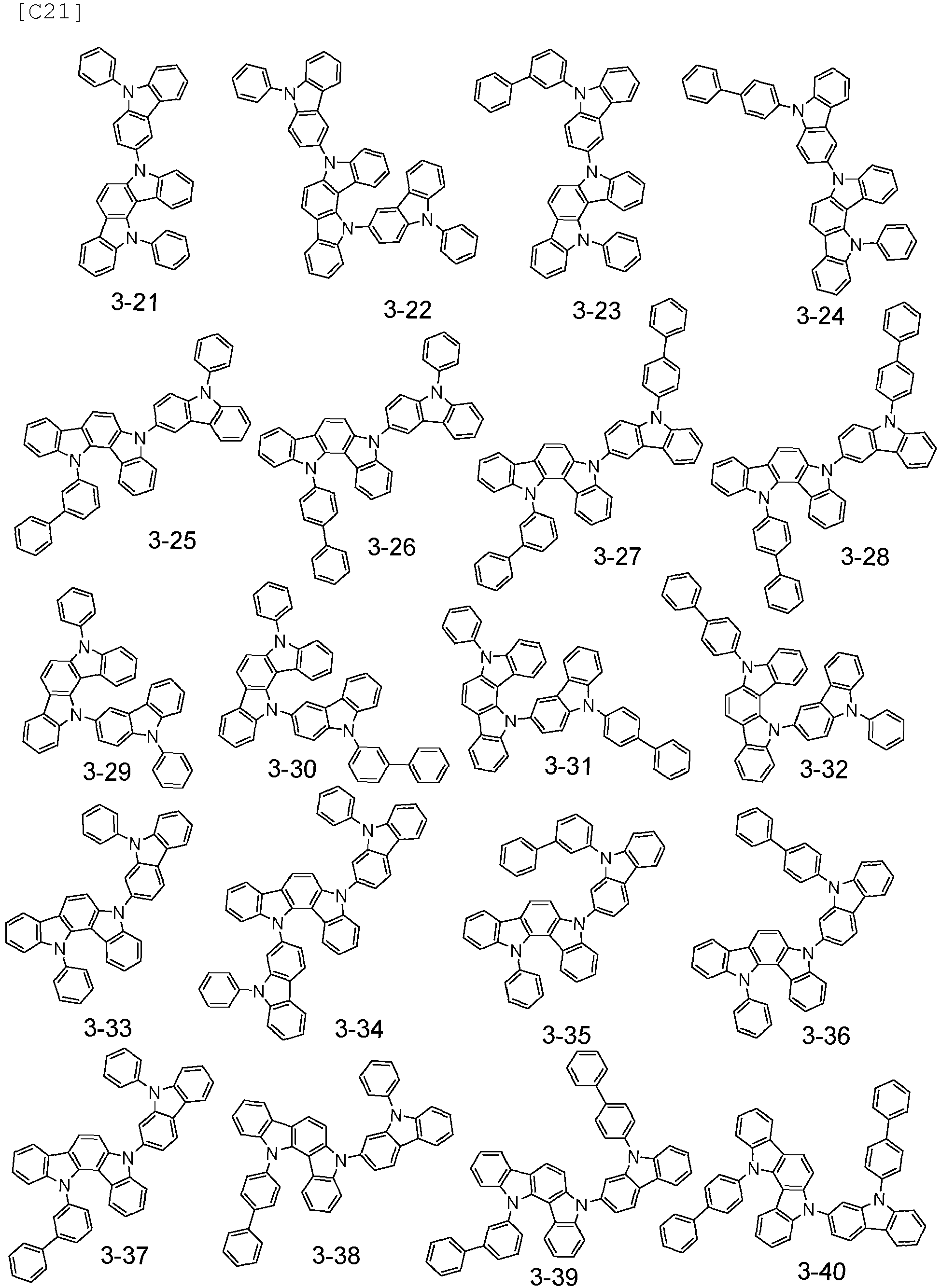

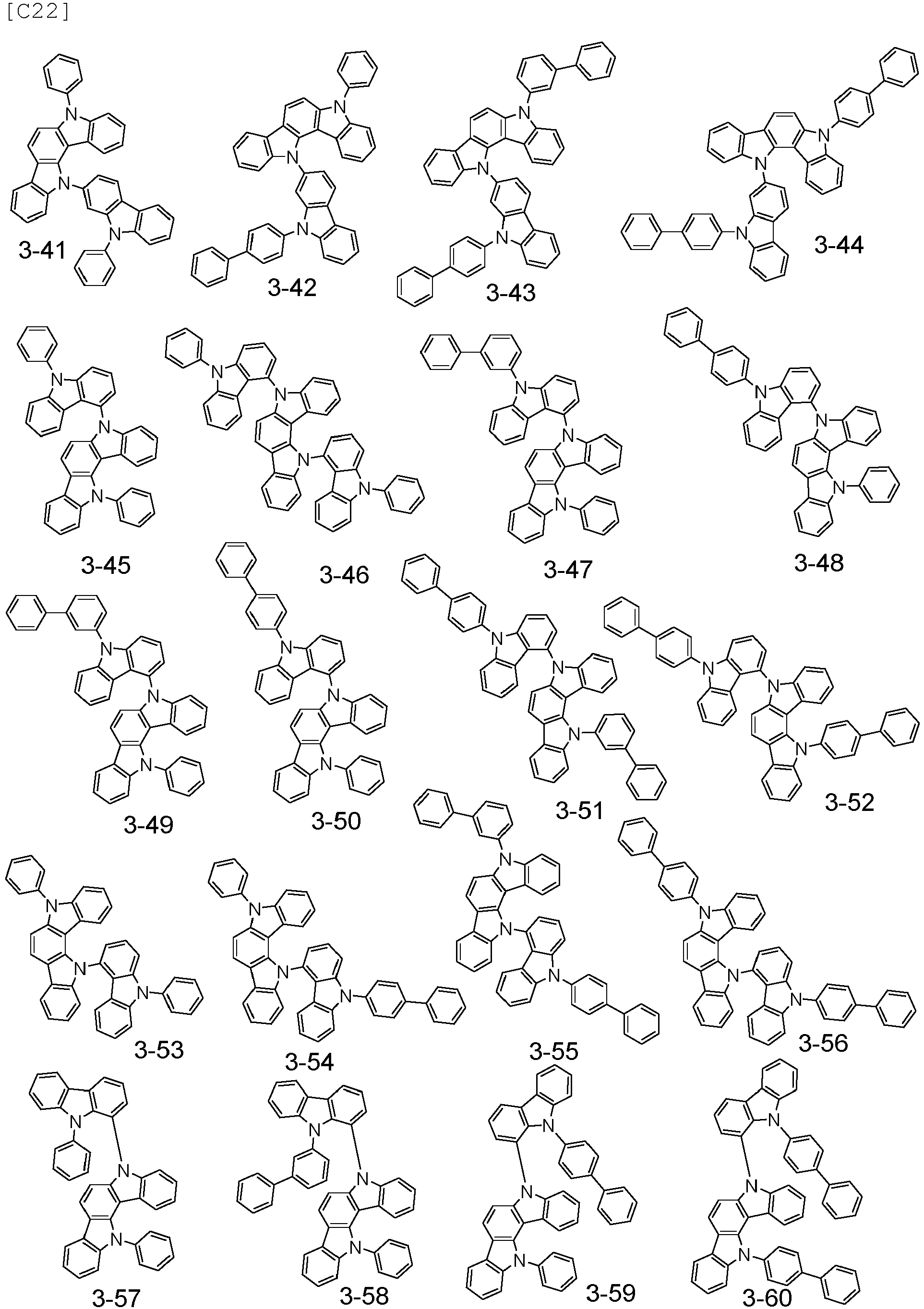

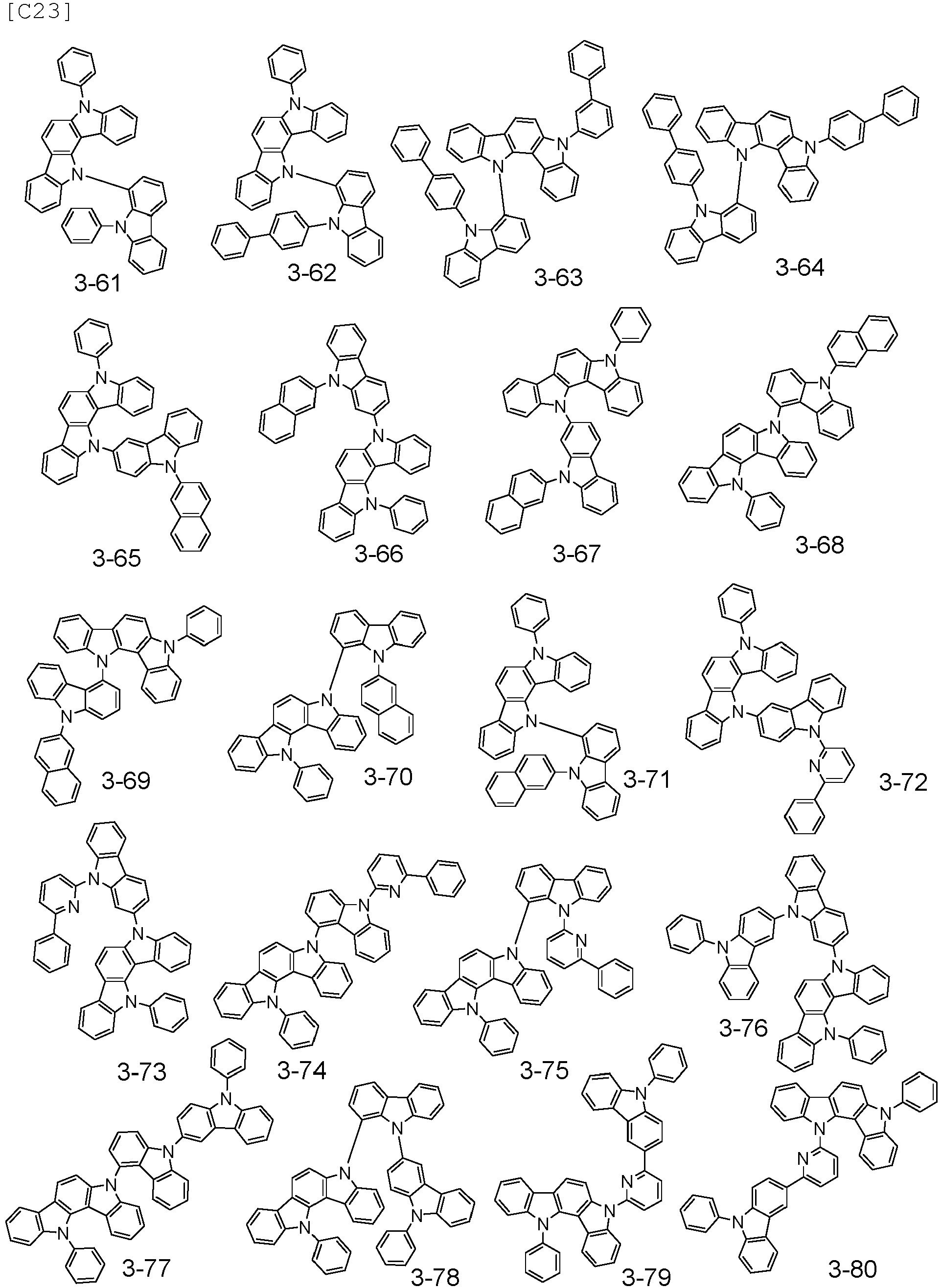

Next, General Formula (4) above will be described.

In General Formula (4), L⁷ is an aromatic hydrocarbon group having 6 to 30 carbon atoms, an aromatic heterocyclic group having 3 to 30 carbon atoms, or a linked aromatic group in which these aromatic rings are linked. The linked aromatic group is a group in which 2 to 10 aromatic rings of an aromatic hydrocarbon group or an aromatic heterocyclic group are linked through direct bonding.

L⁷ is an m-valent group, and the aromatic hydrocarbon group, the aromatic heterocyclic group, or the linked aromatic group may have a substituent.

Specific examples of aromatic hydrocarbon groups or aromatic heterocyclic groups include a group formed by removing m H from such as benzene, pentalene, indene, naphthalene, azulene, heptalene, octalene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, helisene, hexaphene, rubicene, coronene, trinaphthylene, heptaphene, pyranthrene, furan, benzofuran, isobenzofuran, xanthene, oxathrene, dibenzofuran, perixanthenoxanthene, thiophene, thioxanthene, thianthrene, phenoxathiin, thionaphthene, isothianaphthene, thiophanthrene, dibenzothiophene, pyrrole, pyrazole, tetrazole, selenazole, thiazole, isothiazole, oxazole, furazan, indolizine, indole, isoindole, indazole, purine, quinolizine, isoquinoline, imidazole, naphthyridine, phthalazine, benzodiazepine, quinoxaline, cinnoline, quinoline, pteridine, phenanthridine, acridine, perimidine, phenanthroline, phenazine, carboline, phenotellurazine, phenoselenazine, phenothiazine, phenoxazine, anthyridine, benzothiazole, benzoimidazole, benzooxazole, benzoisoxazole, benzoisothiazole, or an aromatic compound or the like in which a plurality of aromatic rings thereof are linked.

In a case where L⁷ is a linked aromatic group, the number of links is preferably 2 to 10 and more preferably 2 to 7, and linked aromatic rings may be the same as or different from each other. In that case, a bonding site for bonding with m carbazolyl groups in General Formula (3) is not limited, but may be a terminal ring or a central ring of linked aromatic rings. Here, aromatic rings collectively mean aromatic hydrocarbon rings and aromatic heterocyclic rings.

Specific examples of the above-described linked aromatic groups include groups formed by removing hydrogen from biphenyl, terphenyl, quaterphenyl, binaphthalene, phenyltriphenylene, phenyldibenzofuran, phenyldibenzothiophene, bisdibenzofuran, bisdibenzothiophene, or the like.

Specific examples of preferred L⁷ include groups formed from benzene, naphthalene, anthracene, biphenyl, terphenyl, dibenzofuran, dibenzothiophene, phenyldibenzofuran, or phenyldibenzothiophene. More preferred examples thereof include groups formed from benzene, biphenyl, or terphenyl.

m represents an integer of 1 to 3. m is preferably 1 or 2 and more preferably 1.

n's are numbers of repetitions and each independently an integer of 1 to 4. n's are preferably 1 to 3. However, at least one n is an integer of 2 to 4.

At least one bonding structure represented by Formula (c1) or (c2) is preferably included in General Formula (4).

All the bonding structures between carbazolyl groups are preferably bonding structures represented by Formula (c1) or (c2) .

The sum of n's (the total number of carbazolyl groups) is an integer of 2 to 12, preferably 2 to 9, and more preferably 2 to 6.

In General Formula (4) and Formulae (c1) and (c2), R's are independently hydrogen, an alkyl group having 1 to 10 carbon atoms, or a cycloalkyl group having 3 to 11 carbon atoms. Hydrogen, an alkyl group having 1 to 8 carbon atoms, or a cycloalkyl group having 3 to 8 carbon atoms is preferable, and hydrogen, an alkyl group having 1 to 4 carbon atoms, or a cycloalkyl group having 5 to 7 carbon atoms is more preferable.

Specific examples of alkyl groups include a methyl group, an ethyl group, a propyl group, a butyl group, a pentyl, a hexyl group, a heptyl group, an octyl group, a nonyl group, and a decyl group, and preferred examples thereof include a methyl group, an ethyl group, a propyl group, a butyl group, a pentyl group, a hexyl group, a heptyl group, and an octyl group. The above-described alkyl groups may be linear or branched.

Specific examples of cycloalkyl groups include a cyclopropyl group, a cyclobutyl group, a cyclopentyl group, a cyclohexyl group, a cycloheptyl group, a cyclooctyl group, and a methylcyclohexyl group, and preferred examples thereof include a cyclohexyl group and a methylcyclohexyl group.

Specific examples of carbazole compounds represented by General Formula (4) will be shown below, but the present invention is not limited thereto.

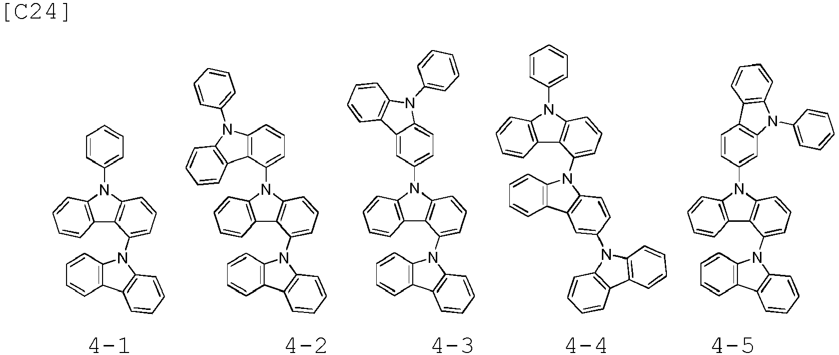

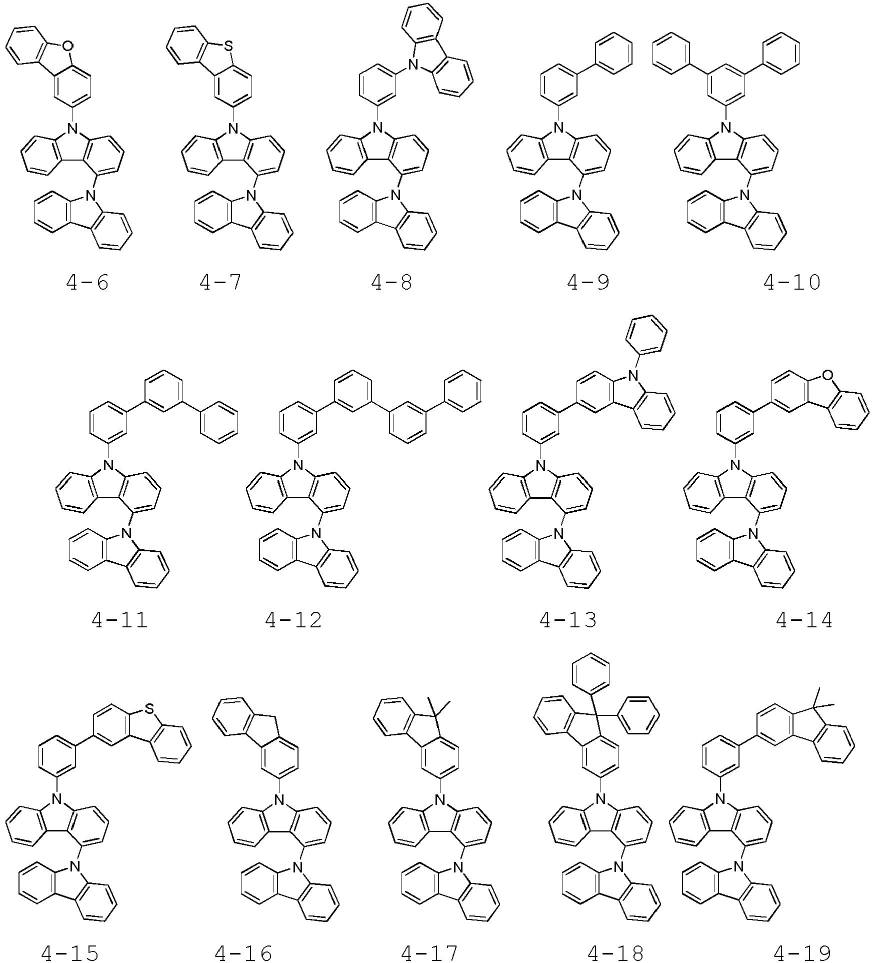

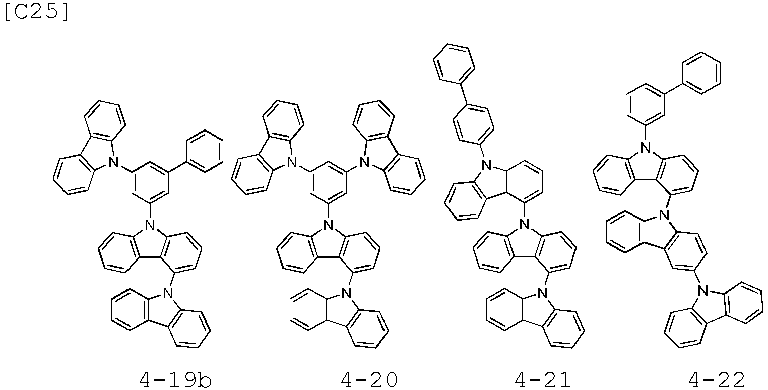

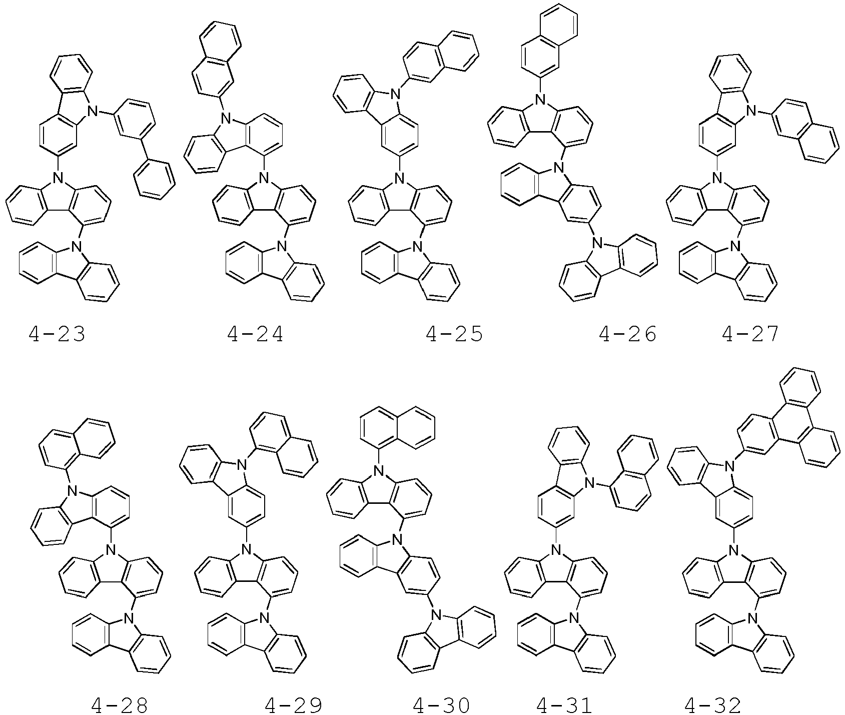

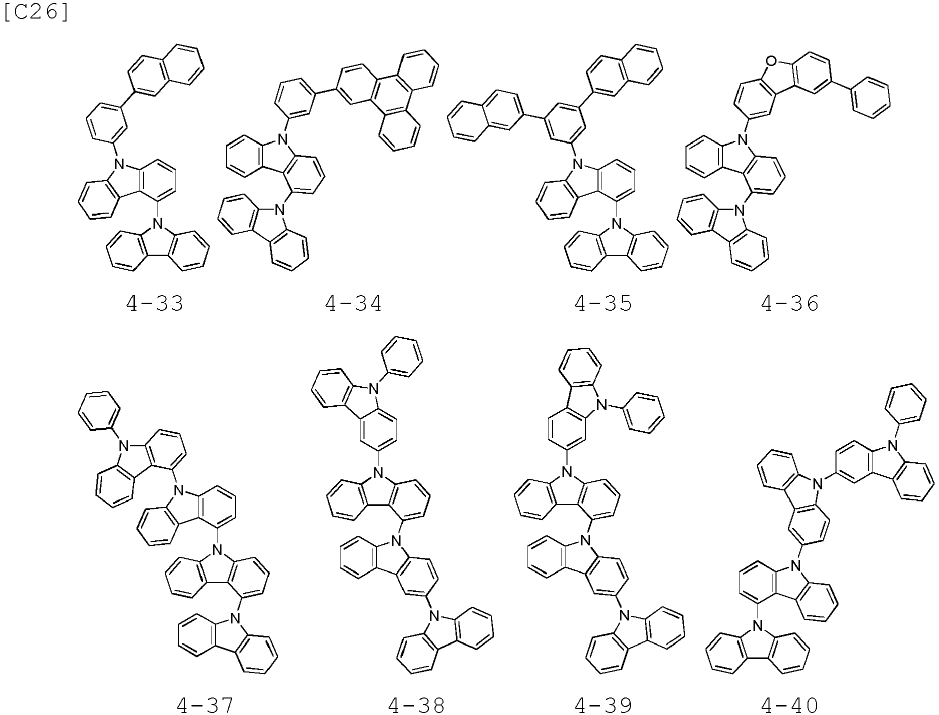

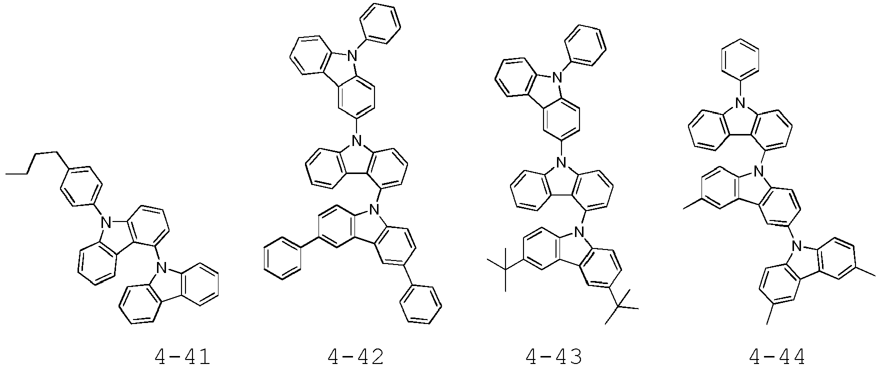

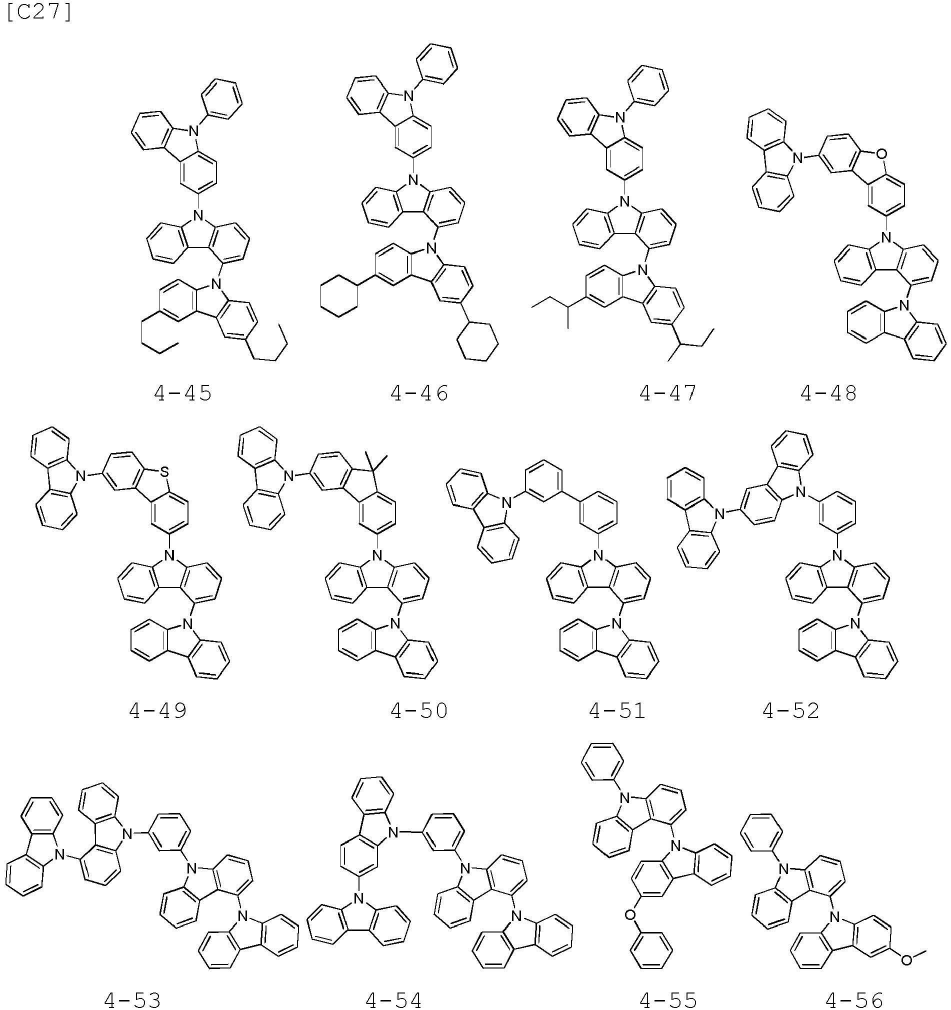

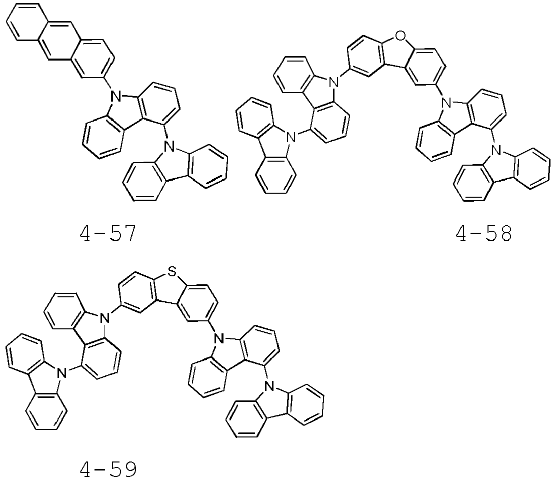

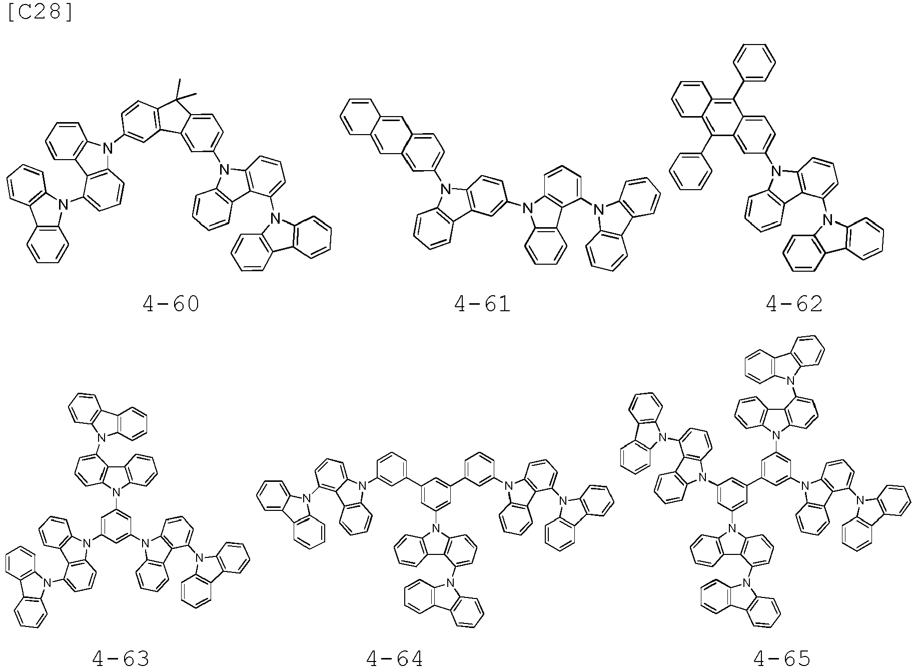

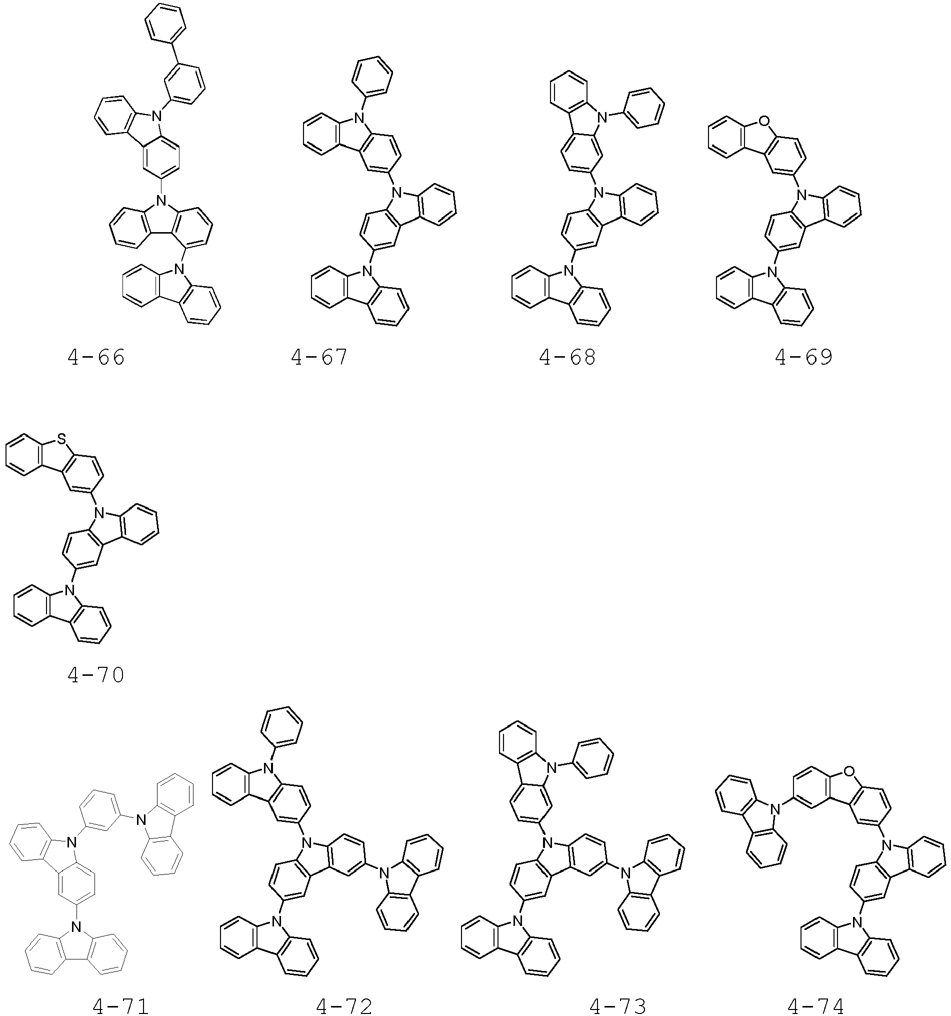

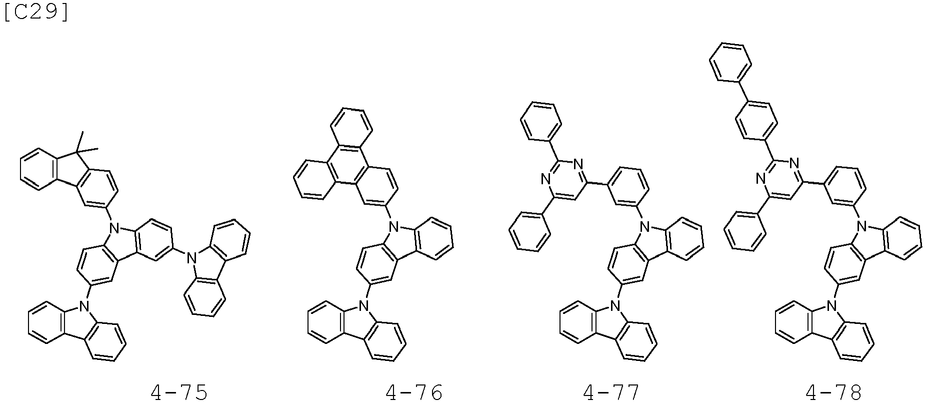

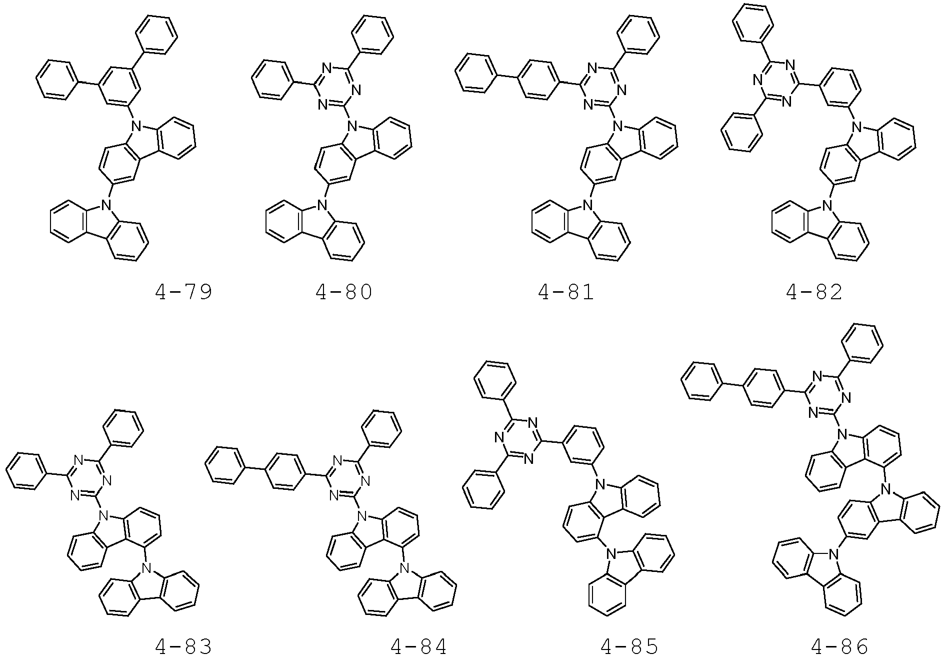

An excellent organic EL device can be provided using a first host selected from the compounds represented by General Formula (1) and a second host selected from the compounds represented by General Formulae (2), (3), or (4) as host materials for a light emitting layer.

The first host and the second host can be used by being vapor-deposited from different vapor deposition sources. However, it is preferable that a premixture be prepared by premixing the first host and the second host before vapor deposition and these be vapor-deposited simultaneously from one vapor deposition source to form a light emitting layer. In this case, a luminescent dopant material required for forming a light emitting layer or other hosts used as necessary may be mixed with this premixture. However, in a case where there is a large difference between temperatures at which desired vapor pressures are obtained, the vapor deposition is preferably performed from different vapor deposition sources.

In addition, regarding the mixing ratio (weight ratio) between a first host and a second host, the proportion of the first host in the total amount of the first host and the second host may be 20% to 60%, and is preferably higher than 20% and lower than 55% and more preferably 40% to 50%.

Next, the structure of the organic EL device of the present invention will be described with reference to the drawings, but is not limited thereto.

Fig. 1 is a cross-sectional view illustrating a structural example of a general organic EL device used in the present invention. 1 represents a substrate, 2 represents an anode, 3 represents a hole injection layer, 4 represents a hole transport layer, 5 represents a light emitting layer, 6 represents an electron transport layer, and 7 represents a cathode. The organic EL device of the present invention may have an exciton-blocking layer adjacent to the light emitting layer or may have an electron-blocking layer between the light emitting layer and the hole injection layer. The exciton-blocking layer can be inserted into the light emitting layer on a cathode side or any position on the cathode side and can be inserted into both sides at the same time. The organic EL device of the present invention has an anode, a light emitting layer, and a cathode as essential layers. However, it is preferable that the organic EL device of the present invention have a hole injection/transport layer and an electron injection/transport layer in addition to the essential layers and further have a hole-blocking layer between the light emitting layer and the electron injection/transport layer. The hole injection/transport layer means either or both of a hole injection layer and a hole transport layer, and the electron injection/transport layer means either or both of an electron injection layer and an electron transport layer.

A structure opposite to that of Fig. 1 can also be used, that is, a cathode 7, an electron transport layer 6, a light emitting layer 5, a hole transport layer 4, and an anode 2 are laminated on a substrate 1 in this order. Even in this case, layers can be added or omitted as necessary.

### -Substrate-

The organic EL device of the present invention is preferably supported by a substrate. Such a substrate is not particularly limited as long as it is conventionally used in organic EL devices, and a substrate made of glass, transparent plastic, quartz, or the like can be used.

### -Anode-

As materials for an anode in an organic EL device, metals, alloys, electrically conductive compounds, or materials composed of a mixture thereof which have a large work function (4 eV or more) are preferably used. Specific examples of such electrode materials include metals such as Au and conductive transparent materials such as Cul, indium tin oxide (ITO), SnO₂, and ZnO. In addition, amorphous materials such as IDIXO (In₂O₃-ZnO) capable of producing a transparent conductive film may be used. Regarding an anode, a thin film may be formed through a method such as vapor deposition or sputtering of these electrode materials to form a pattern having a desired shape through a photolithographic method. Alternatively, in a case where pattern accuracy is not required much (about 100 µm or more), a pattern may be formed using a mask having a desired shape during vapor-depositing or sputtering of the above-described electrode materials. Alternatively, in a case where an applicable substance such as an organic conductive compound is used, wet film formation methods such as a printing method or a coating method can also be used. In a case where light emission is taken out from this anode, it is desirable to increase the transmittance to more than 10% and it is preferable to set the sheet resistance of an anode to several hundred Ω/square or less. The film thickness also depends on materials, but is selected from a range of usually 10 to 1,000 nm and preferably 10 to 200 nm.

### -Cathode-

On the other hand, as cathode materials (electron injecting metals), alloys, electrically conductive compounds, or materials composed of a mixture thereof which have a small work function (4 eV or less) are used. Specific examples of such electrode materials include sodium, sodium-potassium alloys, magnesium, lithium, magnesium-copper mixtures, magnesium-silver mixtures, magnesium-aluminum mixtures, magnesium-indium mixtures, aluminum-aluminum oxide (Al₂O₃) mixtures, indium, lithium-aluminum mixtures, and rare earth metals. Among these, a mixture of an electron injecting metal and a secondary metal which is a stable metal having a larger work function than the electron injecting metal, for example, a magnesium-silver mixture, a magnesium-aluminum mixture, a magnesium-indium mixture, an aluminum-aluminum oxide mixture, a lithium-aluminum mixture, or aluminum is suitable from the viewpoints of electron injecting properties and durability against oxidation. A cathode can be produced by forming a thin film through a method such as vapor deposition or sputtering of these cathode materials. In addition, the sheet resistance of a cathode is preferably several hundred Ω/square or less, and the film thickness is selected from a range of usually 10 nm to 5 µm and preferably 50 to 200 nm. It is preferable that the luminescence be improved by making either an anode or a cathode of an organic EL device be transparent or translucent to allow emitted light to be transmitted therethrough.

In addition, after forming the above-described metals on a cathode at a film thickness of 1 to 20 nm, a conductive transparent material exemplified in the description of the anode can be formed thereon to produce a transparent or translucent cathode. By using this process, a device in which both an anode and a cathode are transparent can be produced.

### -Light Emitting Layer-

A light emitting layer is a layer emitting light after production of excitons due to recombination of holes and electrons respectively injected from an anode and a cathode and contains an organic luminescent dopant material and a host material.

The material for an organic EL device of the present invention can be used for the light emitting layer and can be used as a host material. One kind or two or more kinds of the material for an organic EL devices may be used.

In a case where the material for an organic EL device of the present invention is used as a host material, it is preferably used as a first host and carbazole compounds represented by General Formulae (2) to (4) is preferably used as second hosts.

One kind or two or more kinds of the carbazole compounds represented by General Formulae (2) to (4) may be used.

Furthermore, one kind or two or plural kinds of well-known host materials may be used in combination, and it is preferable that the amount used be 50 wt% or less and preferably 25 wt% or less based on the total amount of the host materials.

The first host and the second host can be vapor-deposited from different vapor deposition sources. Alternatively, a premixture can be prepared by premixing the first host and the second host before vapor deposition and vapor-deposited simultaneously from one vapor deposition source.

In the case where the first host and the second host are used by being premixed, the difference in 50% weight reduction temperature (T₅₀) is desirably small in order to produce an organic EL device having favorable characteristics with good reproducibility. The 50% weight reduction temperature is a temperature when the weight is reduced by 50% when the temperature is raised from room temperature to 550°C at a rate of 10°C per minute in TG-DTA measurement under nitrogen stream decompression (50 Pa). It is thought that vaporization due to evaporation or sublimation occurs most actively in the vicinity of this temperature range.

The difference in 50% weight reduction temperature between the first host and the second host is preferably within 20°C and more preferably within 15°C. As the premixing method, a well-known method such as pulverizing and mixing can be employed, and it is desirable that the mixing be performed as uniformly as possible.

In a case where a phosphorescent dopant is used as a luminescent dopant material, a phosphorescent dopant containing an organic metal complex containing at least one metal selected from the group consisting of ruthenium, rhodium, palladium, silver, rhenium, osmium, iridium, platinum, and gold may be used. Specifically, iridium complexes disclosed in J. Am. Chem. Soc. 2001, 123, 4304 or Japanese Translation of PCT Application No. 2013-53051 are suitably used, but the present invention is not limited thereto.

Only one kind of a phosphorescent dopant material may be contained in a light emitting layer, or two or more kinds of phosphorescent dopant materials may be contained therein. The content of a phosphorescent dopant material with respect to a host material is preferably 0.1 to 30 wt% and more preferably 1 to 20 wt%.

The phosphorescent dopant material is not particularly limited, but specific examples thereof include the following.

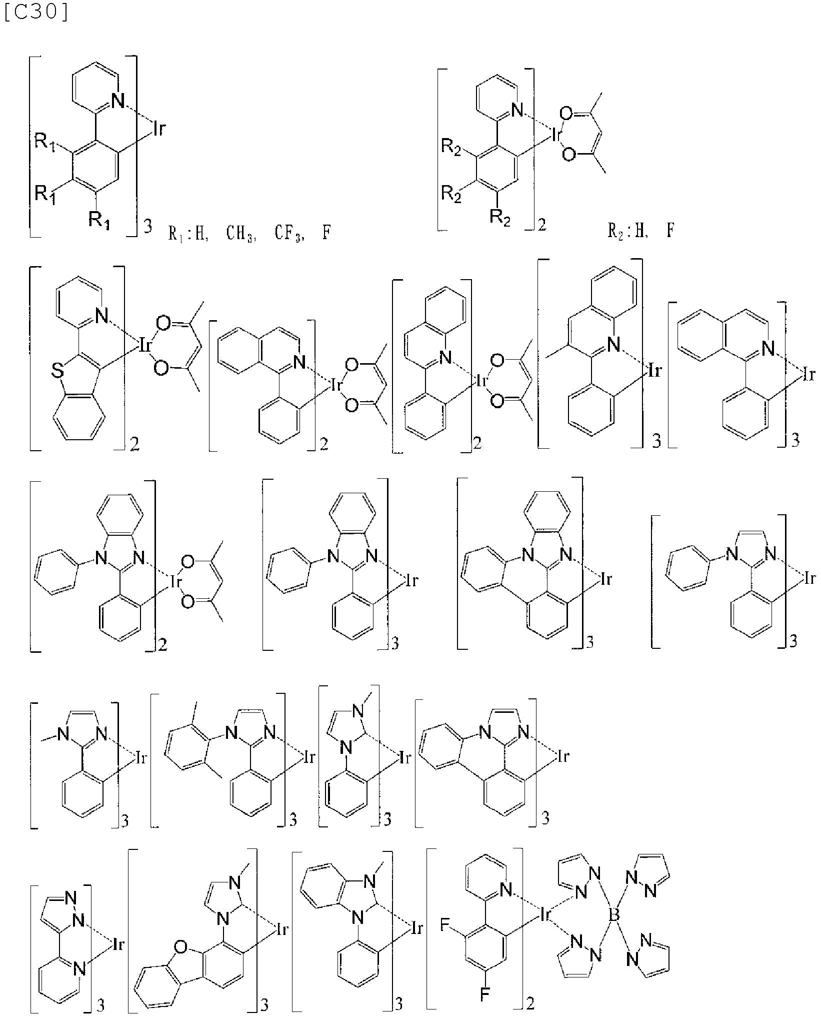

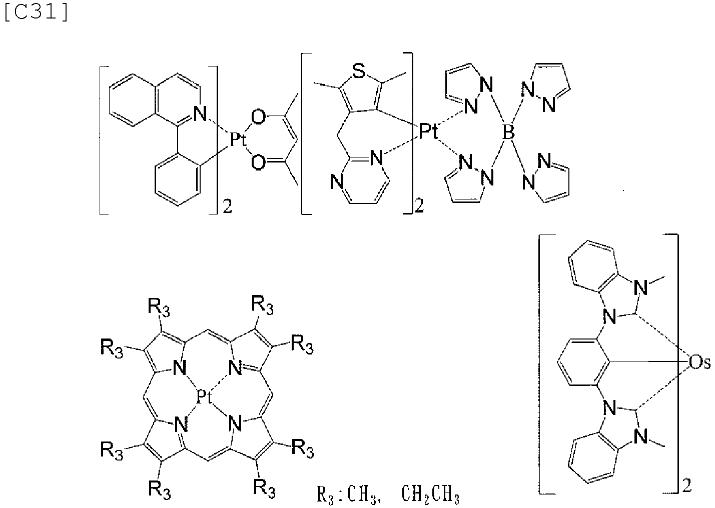

In a case where a fluorescent dopant is used as a luminescent dopant material, the fluorescent dopant is not particularly limited, but examples thereof include benzoxazole derivatives, benzothiazole derivatives, benzoimidazole derivatives, styrylbenzene derivatives, polyphenyl derivatives, diphenylbutadiene derivatives, tetraphenylbutadiene derivatives, naphthalimide derivatives, coumarin derivatives, condensed aromatic compounds, perinone derivatives, oxadiazole derivatives, oxazine derivatives, aldazine derivatives, pyrrolidine derivatives, cyclopentadiene derivatives, bisstyrylanthracene derivatives, quinacridone derivatives, pyrrolopyridine derivatives, thiadiazolopyridine derivatives, styrylamine derivatives, diketopyrrolopyrrole derivatives, aromatic dimethylidyne compounds, various metal complexes typified by metal complexes of 8-quinolinol derivatives, metal complexes of pyrromethene derivatives, rare earth complexes, and transition metal complexes, polymer compounds such as polythiophene, polyphenylene, and polyphenylene vinylene, and organic silane derivatives. Preferred examples thereof include condensed aromatic derivatives, styryl derivatives, diketopyrrolopyrrole derivatives, oxazine derivatives, pyrromethene metal complexes, transition metal complexes, and lanthanoid complexes, and more preferred examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo [a] naphthalene, hexacene, naphth[2,1-f]isoquinoline, α-naphthaphenanthridin, phenanthrooxazole, quinolino[6,5-f]quinoline, and benzothiophanthrene. These may have an alkyl group, an aryl group, an aromatic heterocyclic group, or a diarylamino group as a substituent.

Only one kind of a fluorescent dopant material may be contained in a light emitting layer, or two or more kinds of phosphorescent dopant materials may be contained therein. The content of a fluorescent dopant material with respect to a host material is preferably 0.1 to 20 % and more preferably 1 to 10 %.

In a case where a thermally activated delayed fluorescent dopant is used as a luminescent dopant material, although the thermally activated delayed fluorescent dopant is not particularly limited, examples thereof include metal complexes such as a tin complex or a copper complex, indolocarbazole derivatives disclosed in WO2011/070963, cyanobenzene derivatives and carbazole derivatives disclosed in Nature 2012, 492, 234, and phenazine derivatives, oxadiazole derivatives, triazole derivatives, sulfone derivatives, phenoxazine derivatives, and acridine derivatives disclosed in Nature Photonics 2014, 8, 326.

The thermally activated delayed fluorescent dopant material is not particularly limited, but specific examples thereof include the following.

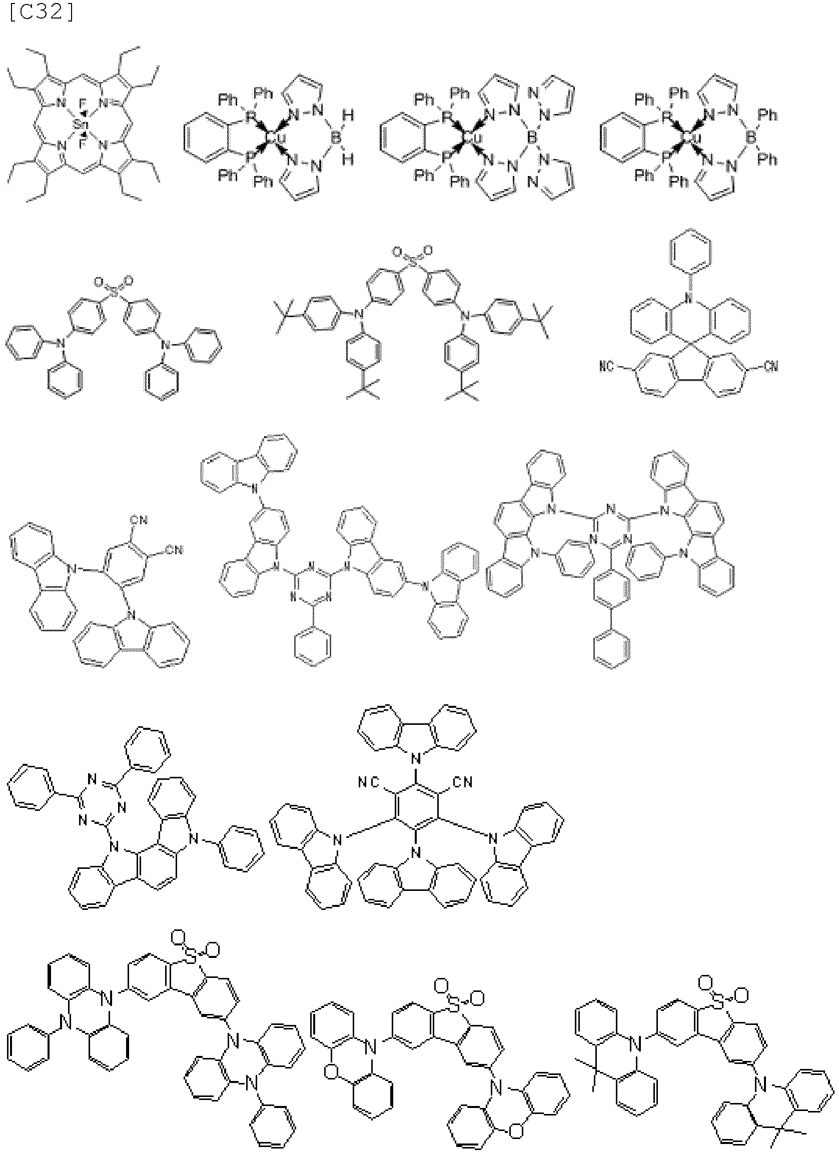

Only one kind of a thermally activated delayed fluorescent dopant material may be contained in a light emitting layer, or two or more kinds of thermally activated delayed fluorescent dopant materials may be contained therein. In addition, the thermally activated delayed fluorescent dopant may be used by being mixed with a phosphorescent dopant or a fluorescent dopant. The content of a thermally activated delayed fluorescent dopant material with respect to a host material is preferably 0.1 to 50 % and more preferably 1 to 30 %.

### -Injection Layer-

An injection layer is a layer, such as a hole injection layer or an electron injection layer, provided between an electrode and an organic layer for reducing a drive voltage or improving luminescence and may be present between an anode and a light emitting layer or a hole transport layer and between a cathode and a light emitting layer or an electron transport layer. The injection layer can be provided as necessary.

### -Hole-Blocking Layer-

A hole-blocking layer has a function of an electron transport layer in a broad sense and is made of a hole-blocking material which has a function of transporting electrons and a significantly low ability of transporting holes. By blocking holes while transporting electrons, the probability of recombining electrons and holes in a light emitting layer can be increased.

A well-known hole-blocking layer material can be used in a hole-blocking layer, but the compound represented by General Formula (1) is preferably contained in a hole-blocking layer.

### -Electron-Blocking Layer-

An electron-blocking layer has a function of a hole transport layer in a broad sense. By blocking electrons while transporting holes, the probability of recombining electrons and holes in a light emitting layer can be increased.

A well-known electron-blocking layer material can be used as the electron-blocking layer material, and a hole transport layer material to be described below can be used as necessary. The film thickness of an electron-blocking layer is preferably 3 to 100 nm and more preferably 5 to 30 nm.

### -Exciton-Blocking Layer-

An exciton-blocking layer is a layer for blocking excitons generated by recombination of holes and electrons in a light emitting layer from being diffused in a charge transport layer. When this exciton-blocking layer is inserted, excitons can be efficiently confined in a light emitting layer and the luminous efficiency of an device can be improved. In a case of an device in which two or more light emitting layers are adjacent, an exciton-blocking layer can be inserted between the two adjacent light emitting layers.

A well-known exciton-blocking layer material can be used as the exciton-blocking layer material. Examples thereof include 1,3-dicarbazolylbenzene (mCP) and bis(2-methyl-8-quinolinolato)-4-phenylphenolato aluminum (III) (BAlq).

### -Hole Transport Layer-

A hole transport layer is made of a hole transport material having a function of transporting holes, and a single hole transport layer or a plurality of hole transport layers can be provided.

As a hole transport material, one which is either an organic substance or an inorganic substance having either the ability of injecting or transporting holes or electron barrier properties may be used. An arbitrary compound selected from conventionally well-known compounds can be used in a hole transport layer. Examples of such hole transport materials include porphyrin derivatives, arylamine derivatives, triazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, oxazole derivatives, styrylanthracene derivatives, fluorenone derivatives, hydrazone derivatives, stilbene derivatives, silazane derivatives, aniline copolymers, and conductive polymer oligomers, and especially thiophene oligomers. Porphyrin derivatives, arylamine derivatives, and styrylamine derivatives are preferably used, and arylamine compounds are more preferably used.

### -Electron Transport Layer-

An electron transport layer is made of a material having a function of transporting electrons, and a single electron transport layer or a plurality of electron transport layers can be provided.

An electron transport material (which may also serve as a hole-blocking material) may have a function of transmitting electrons injected from a cathode to a light emitting layer. An arbitrary compound selected from conventionally well-known compounds can be used in an electron transport layer, and examples thereof include polycyclic aromatic derivatives such as naphthalene, anthracene, and phenanthroline, tris(8-quinolinolato)aluminum (III) derivatives, phosphine oxide derivatives, nitro-substituted fluorene derivatives, diphenylquinone derivatives, thiopyran dioxide derivatives, carbodiimide, fluorenylidene methane derivatives, anthraquinodimethane, anthrone derivatives, bipyridine derivatives, quinoline derivatives, oxadiazole derivatives, benzoimidazole derivatives, benzothiazole derivatives, and indolocarbazole derivatives. Furthermore, polymer materials in which these materials are introduced into polymer chains or used as polymer main chains can also be used. Examples

Hereinafter, the present invention will be described in more detail with reference to examples. However, as a matter of course, the present invention is not limited to these examples and can be carried out in various forms as long as it does not exceed the gist thereof.

The compound represented by General Formula (1) used in the present invention was synthesized through the route shown below. Compound numbers correspond to the numbers assigned to the above-described chemical formulas.

### Example 1G

### Synthesis of Compound 1-1

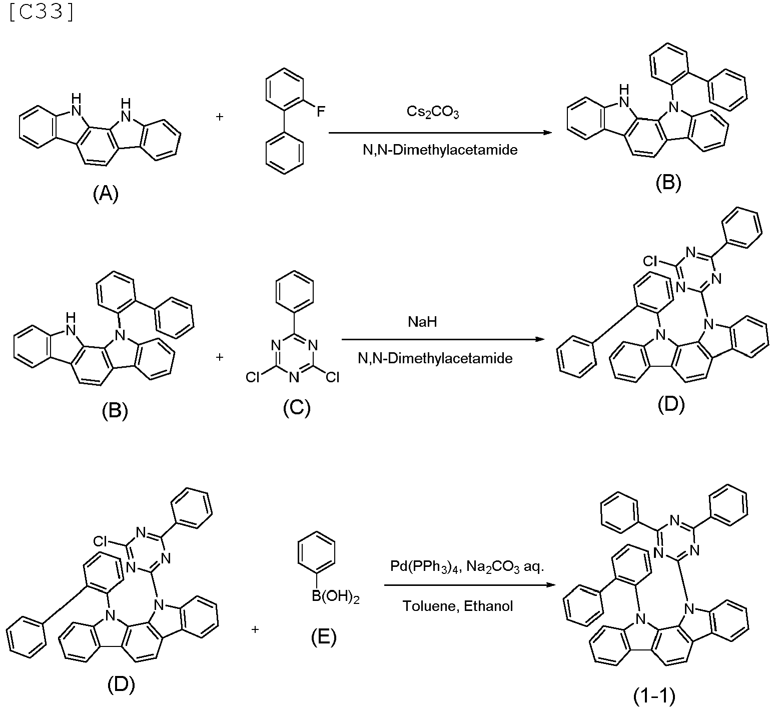

20.0 g (78.0 mmol) of a compound A, 14.8 g (85.8 mmol) of 2-fluorobiphenyl, 50.8 g (156 mmol) of cesium carbonate, and 500 ml of N,N-dimethylacetamide were added in a nitrogen atmosphere and stirred overnight while heating at 190°C. The reaction solution was cooled at room temperature, and then, distilled water (500 ml) was added thereto with stirring and precipitated solids were filtered. The obtained solids were subjected to silica gel column chromatography to obtain 25.4 g (62.4 mmol, yield 80.0%) of a compound B as white solids.

1.1 g (27.0 mol) of sodium hydride (62.0%) and 10 ml of dehydrated N,N-dimethylacetamide (DMAc) were added in a nitrogen atmosphere and stirred at room temperature for 0.5 hours. A DMAc (300 ml) solution with 10.0 g (24.5 mmol) of the compound B was added to the obtained suspension, and the mixture was stirred at room temperature for 30 minutes. 6.44 g (29.4 mmol) of a compound C was added to the obtained suspension, and the mixture was stirred at room temperature for 0.5 hours. Distilled water (500 ml) was added to the obtained suspension with stirring, and precipitated solids were filtered. The obtained solids were subjected to silica gel column chromatography to obtain 13.4 g (22.0 mmol, yield 89.8%) of a compound D as white solids.

10.0 g (16.7 mmol) of the compound D, 2.44 g (20.1 mmol) of phenylboronic acid, 0.966 g (0.836 mmol) of tetrakis(triphenylphosphine)palladium(0), an aqueous solution (16.7 ml) with 3.54 g of sodium carbonate, 100 ml of toluene, and 20 ml of ethanol were added in a nitrogen atmosphere and stirred for 1 hour while heating at 90°C. The reaction solution was cooled at room temperature, and then, 200 ml of toluene and distilled water (200 ml) were added thereto with stirring. The organic layer was washed with distilled water (3 × 200 ml). After the organic layer was dried with anhydrous magnesium sulfate, magnesium sulfate was filtered and the solvent was distilled off under reduced pressure. The obtained residue was purified through silica gel column chromatography and subjected to crystallization and purification to obtain 5.23 g (8.17 mol, yield of 49.0%) of a compound 1-1 (APCI-TOFMS, m/z 540 [M+H]⁺) as white solids.

### Example 2G

### Synthesis of Compound 1-3

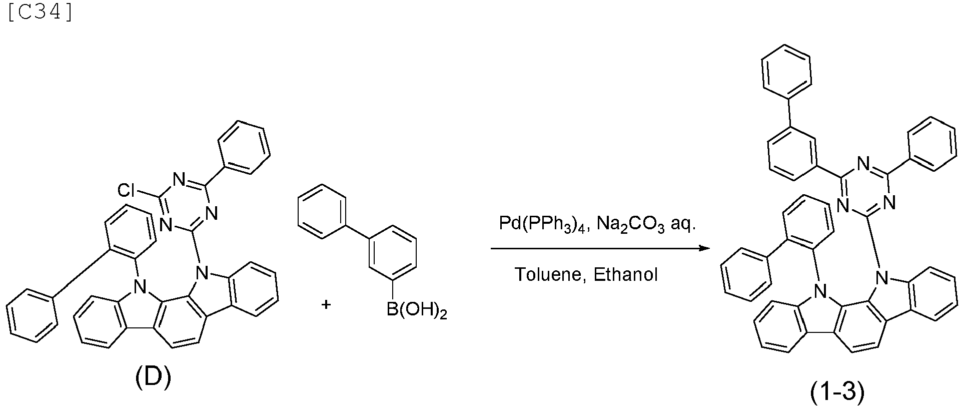

10.0 g (16.7 mmol) of the compound D, 3.98 g (20.1 mmol) of 3-biphenylboronic acid, 0.971 g (0.840 mmol) of tetrakis(triphenylphosphine)palladium(0), an aqueous solution (18.2 ml) with 3.54 g of sodium carbonate, 100 ml of toluene, and 20 ml of ethanol were added in a nitrogen atmosphere and stirred for 1 hour while heating at 90°C. The reaction solution was cooled at room temperature, and then, 200 ml of toluene and distilled water (200 ml) were added thereto with stirring. The organic layer was washed with distilled water (3 × 200 ml). After the organic layer was dried with anhydrous magnesium sulfate, magnesium sulfate was filtered and the solvent was distilled off under reduced pressure. The obtained residue was purified through silica gel column chromatography and subjected to crystallization and purification to obtain 5.49 g (7.67 mmol, yield of 45.9%) of a compound 1-3 (APCI-TOFMS, m/z 716 [M+H]⁺) as white solids.

### Example 3G

### Synthesis of Compound 1-4

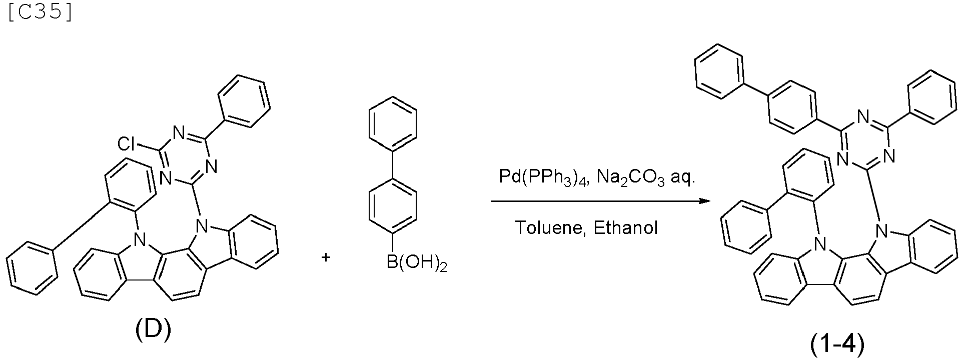

10.0 g (16.7 mmol) of the compound D, 3.98 g (20.1 mmol) of 4-biphenylboronic acid, 0.960 g (0.835 mmol) of tetrakis(triphenylphosphine)palladium(0), an aqueous solution (17.0 ml) with 3.54 g of sodium carbonate, 200 ml of toluene, and 20 ml of ethanol were added in a nitrogen atmosphere and stirred for 1 hour while heating at 90°C. The reaction solution was cooled at room temperature, and then, 400 ml of toluene and distilled water (200 ml) were added thereto with stirring. The organic layer was washed with distilled water (3 × 400 ml). After the organic layer was dried with anhydrous magnesium sulfate, magnesium sulfate was filtered and the solvent was distilled off under reduced pressure. The obtained residue was purified through silica gel column chromatography and subjected to crystallization and purification to obtain 5.91 g (8.26 mmol, yield of 49.4%) of a compound 1-4 (APCI-TOFMS, m/z 716 [M+H]⁺) as white solids.

### Example 4G

### Synthesis of Compound 1-11

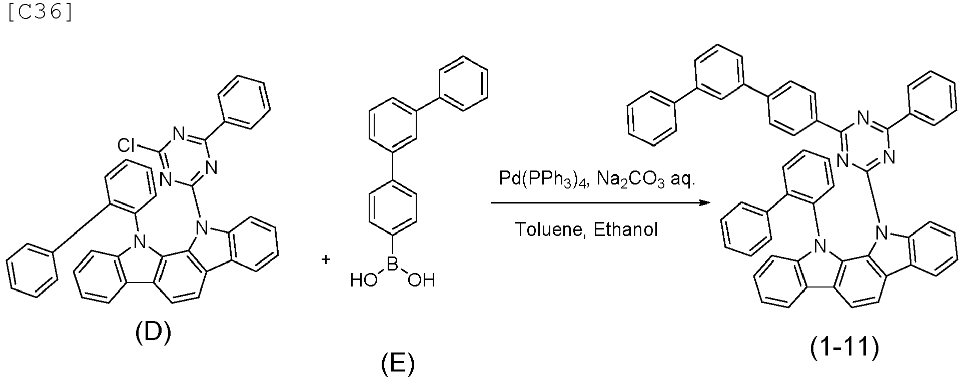

13.4 g (22.0 mmol) of the compound D, 8.60 g (24.2 mmol) of a compound E, 1.27 g (1.10 mmol) of tetrakis(triphenylphosphine)palladium(0), an aqueous solution (35.0 ml) with 7.00 g of sodium carbonate, 200 ml of toluene, and 40 ml of ethanol were added in a nitrogen atmosphere and stirred for 1 hour while heating at 90°C. The reaction solution was cooled at room temperature, and then, 400 ml of toluene and distilled water (200 ml) were added thereto with stirring. The organic layer was washed with distilled water (3 × 400 ml). After the organic layer was dried with anhydrous magnesium sulfate, magnesium sulfate was filtered and the solvent was distilled off under reduced pressure. The obtained residue was purified through silica gel column chromatography and subjected to crystallization and purification to obtain 6.10 g (7.58 mmol, yield of 34.5%) of a compound 1-11 (APCI-TOFMS, m/z 792 [M+H]⁺) as white solids.

### Example 5G

### Synthesis of Compound 1-20

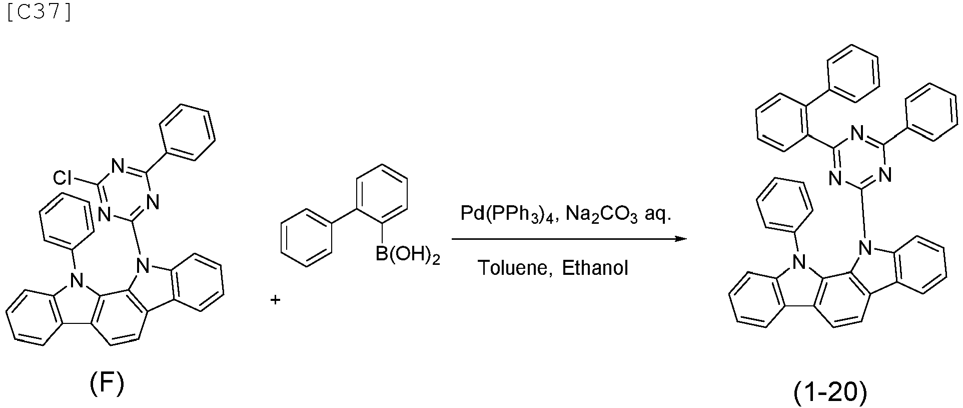

10.0 g (19.2 mmol) of a compound F, 4.55 g (23.0 mmol) of 2-biphenylboronic acid, 1.11 g (0.960 mmol) of tetrakis(triphenylphosphine)palladium(0), an aqueous solution (19.5 ml) with 4.07 g of sodium carbonate, 150 ml of toluene, and 20 ml of ethanol were added in a nitrogen atmosphere and stirred overnight while heating at 90°C. The reaction solution was cooled at room temperature, and then, 200 ml of toluene and distilled water (200 ml) were added thereto with stirring. The organic layer was washed with distilled water (3 × 200 ml). After the organic layer was dried with anhydrous magnesium sulfate, magnesium sulfate was filtered and the solvent was distilled off under reduced pressure. The obtained residue was purified through silica gel column chromatography and subjected to crystallization and purification to obtain 6.26 g (9.78 mmol, yield of 50.9%) of a compound 1-20 (APCI-TOFMS, m/z 640 [M+H]⁺) as white solids.

Compounds 1-24, 1-27, 1-35, 1-37, 1-52, 1-63, 1-68, and 1-76 were synthesized according to the synthesis method in the above-described Examples 1G to 5G and used for production of organic EL devices.

### Example 1

Each thin film was laminated on a glass substrate, on which an anode made of ITO and having a film thickness of 110 nm was formed, with a vacuum degree of 4.0 × 10⁻⁵ Pa through a vacuum vapor deposition method. First, 25 nm thick HAT-CN was formed on the ITO as a hole injection layer, and then 30 nm thick NPD was formed thereon as a hole transport layer. Next, 10 nm thick HT-1 was formed thereon as an electron-blocking layer. Next, a compound 1-1 as a host and Ir(ppy)₃ as a luminescent dopant were subjected to co-vapor deposition from different vapor deposition sources to form a light emitting layer having a thickness of 40 nm. At this time, the co-vapor deposition was performed under the vapor deposition condition where the concentration of Ir(ppy)₃ was 10 wt%. Next, 20 nm thick ET-1 was formed thereon as an electron transport layer. Furthermore, 1 nm thick LiF was formed on the electron transport layer as an electron injection layer. Finally, 70 nm thick Al was formed on the electron injection layer as a cathode to produce an organic EL device.

### Examples 2 to 6

Organic EL devices were produced in the same manner as in Example 1 except that each compound shown in Table 1 was used as a host.

### Example 7

Each thin film was laminated on a glass substrate, on which an anode made of ITO and having a film thickness of 110 nm was formed, with a vacuum degree of 4.0 × 10⁻⁵ Pa through a vacuum vapor deposition method. First, 25 nm thick HAT-CN was formed on the ITO as a hole injection layer, and then 30 nm thick NPD was formed thereon as a hole transport layer. Next, 10 nm thick HT-1 was formed thereon as an electron-blocking layer. Next, a compound 1-4 as a first host, a compound 2-1 as a second host, and Ir(ppy)₃ as a luminescent dopant were subjected to co-vapor deposition from different vapor deposition sources to form a light emitting layer having a thickness of 40 nm. At this time, the co-vapor deposition was performed under the vapor deposition conditions where the concentration of Ir(ppy)₃ was 10 wt% and the weight ratio of the first host to the second host was 30:70. Next, 20 nm thick ET-1 was formed thereon as an electron transport layer. Furthermore, 1 nm thick LiF was formed on the electron transport layer as an electron injection layer. Finally, 70 nm thick Al was formed on the electron injection layer as a cathode to produce an organic EL device.

### Examples 8 to 175

Organic EL devices were produced in the same manner as in Example 7 except that each compound shown in Tables 1 to 7 was used as a first host and a second host.

### Examples 176 to 193

A first host and a second host were mixed with each other in advance to prepare a premixture and subjected to co-vapor deposition from one vapor deposition source.

A first host (0.30 g) and a second host (0.70 g) in Example 7 were weighed and mixed with each other while being ground in a mortar to obtain a premixture. Organic EL devices were produced in the same manner as in Example 7 except that this premixture was used.

### Examples 194 to 202

A first host and a second host were mixed with each other in advance to prepare a premixture and subjected to co-vapor deposition from one vapor deposition source, a hole-blocking layer in which a compound 1-4 was used was further provided thereon.

Organic EL devices were obtained in the same manner as in Examples 176 to 193 except that after a light emitting layer was formed, a 10 nm thick compound 1-4 was formed thereon as a hole-blocking layer, and 10 nm thick ET-1 was formed as an electron transport layer.

Evaluation results of the produced organic EL devices are shown in Tables 1 to 8. In the tables, the Luminance, the drive voltage, and the luminous efficiency are values when the drive current is 20 mA/cm² and are initial characteristics. LT70 is the time required for the initial luminance to attenuate to 70% and represents lifespan characteristics.

**[Table 1]**

| Ex. | First host compd. | Second host compd. | Luminance (cd/m²) | Voltage (V) | Power efficiency (lm/W) | LT70 (h) |
|---|---|---|---|---|---|---|
| 1 | 1-1 | - | 7000 | 3.3 | 33.3 | 700 |
| 2 | 1-3 | - | 7100 | 3.3 | 33.8 | 700 |
| 3 | 1-4 | - | 7000 | 3.2 | 34.4 | 850 |
| 4 | 1-11 | - | 7000 | 3.2 | 34.4 | 800 |
| 5 | 1-20 | - | 7000 | 3.3 | 33.3 | 700 |
| 6 | 1-24 | - | 7000 | 3.1 | 35.5 | 800 |
| 7 | 1-4 | 2-1 | 10200 | 4.2 | 38.1 | 900 |
| 8 | 1-4 | 2-2 | 10300 | 3.9 | 41.5 | 1100 |
| 9 | 1-4 | 2-3 | 10300 | 3.9 | 41.5 | 1150 |
| 10 | 1-4 | 2-5 | 10200 | 3.9 | 41.1 | 900 |
| 11 | 1-4 | 2-27b | 10200 | 3.9 | 41.1 | 1000 |
| 12 | 1-4 | 2-31b | 10200 | 3.9 | 41.1 | 1000 |
| 13 | 1-4 | 2-32b | 10200 | 3.9 | 41.1 | 950 |
| 14 | 1-4 | 2-33b | 10300 | 4.0 | 40.4 | 1200 |
| 15 | 1-4 | 2-37 | 10400 | 4.0 | 40.8 | 950 |
| 16 | 1-4 | 3-1 | 10000 | 3.9 | 40.3 | 900 |
| 17 | 1-4 | 3-4 | 10000 | 3.9 | 40.3 | 900 |
| 18 | 1-4 | 3-9 | 10000 | 3.9 | 40.3 | 900 |
| 19 | 1-4 | 3-17 | 10000 | 3.9 | 40.3 | 900 |
| 20 | 1-4 | 3-19 | 10000 | 3.9 | 40.3 | 950 |
| 21 | 1-4 | 3-21 | 10000 | 3.8 | 41.3 | 1000 |
| 22 | 1-4 | 3-23 | 10000 | 3.8 | 41.3 | 1000 |
| 23 | 1-4 | 3-24 | 10000 | 3.9 | 40.3 | 1000 |
| 24 | 1-4 | 3-28 | 10000 | 3.8 | 41.3 | 1000 |
| 25 | 1-4 | 3-33 | 10000 | 3.9 | 40.3 | 1000 |
| 26 | 1-4 | 3-36 | 10000 | 3.8 | 41.3 | 1000 |
| 27 | 1-4 | 3-45 | 10000 | 3.9 | 40.3 | 950 |
| 28 | 1-4 | 3-48 | 10000 | 3.8 | 41.3 | 1050 |
| 29 | 1-4 | 3-57 | 10000 | 3.9 | 40.3 | 900 |

**[Table 2]**

| Ex. | First host compd. | Second host compd. | Luminance (cd/m²) | Voltage (V) | Power efficiency (lm/W) | LT70 (h) |
|---|---|---|---|---|---|---|
| 30 | 1-4 | 4-2 | 10200 | 3.9 | 41.1 | 900 |
| 31 | 1-4 | 4-3 | 10200 | 4.0 | 40.1 | 1000 |
| 32 | 1-4 | 4-4 | 10200 | 4.0 | 40.1 | 1000 |
| 33 | 1-4 | 4-6 | 10200 | 4.0 | 40.1 | 900 |
| 34 | 1-4 | 4-10 | 10200 | 4.0 | 40.1 | 950 |
| 35 | 1-4 | 4-13 | 10200 | 4.0 | 40.1 | 1000 |
| 36 | 1-4 | 4-14 | 10200 | 4.0 | 40.1 | 1000 |
| 37 | 1-4 | 4-19b | 10200 | 4.0 | 40.1 | 1000 |
| 38 | 1-4 | 4-32 | 10200 | 4.0 | 40.1 | 1000 |
| 39 | 1-4 | 4-67 | 10300 | 4.1 | 39.5 | 1200 |
| 40 | 1-4 | 4-69 | 10300 | 4.1 | 39.5 | 1150 |
| 41 | 1-4 | 4-74 | 10300 | 4.1 | 39.5 | 1000 |
| 42 | 1-4 | 4-81 | 10300 | 4.1 | 39.5 | 1000 |
| 43 | 1-4 | 4-84 | 10300 | 4.1 | 39.5 | 1000 |
| 44 | 1-1 | 2-2 | 10300 | 4.0 | 40.4 | 1000 |
| 45 | 1-1 | 2-3 | 10400 | 4.0 | 40.8 | 1000 |
| 46 | 1-1 | 2-33b | 10400 | 3.9 | 41.9 | 1100 |
| 47 | 1-1 | 2-37 | 10400 | 4.0 | 40.8 | 900 |
| 48 | 1-1 | 3-21 | 10100 | 3.9 | 40.7 | 950 |
| 49 | 1-1 | 3-33 | 10100 | 3.9 | 40.7 | 950 |
| 50 | 1-1 | 3-45 | 10100 | 4.0 | 39.7 | 900 |
| 51 | 1-1 | 3-57 | 10100 | 3.9 | 40.7 | 900 |
| 52 | 1-1 | 4-3 | 10300 | 4.1 | 39.5 | 950 |
| 53 | 1-1 | 4-4 | 10300 | 4.1 | 39.5 | 950 |
| 54 | 1-1 | 4-67 | 10400 | 4.2 | 38.9 | 1100 |
| 55 | 1-1 | 4-69 | 10400 | 4.2 | 38.9 | 1000 |

**[Table 3]**

| Ex. | First host compd. | Second host compd. | Luminance (cd/m²) | Voltage (V) | Power efficiency (lm/W) | LT70 (h) |
|---|---|---|---|---|---|---|
| 56 | 1-11 | 2-2 | 10300 | 4.0 | 40.4 | 1100 |
| 57 | 1-11 | 2-3 | 10300 | 4.1 | 39.5 | 1100 |
| 58 | 1-11 | 2-33b | 10300 | 4.1 | 39.5 | 1200 |
| 59 | 1-11 | 2-37 | 10300 | 3.9 | 41.5 | 1000 |
| 60 | 1-11 | 3-21 | 10100 | 3.6 | 44.1 | 1000 |
| 61 | 1-11 | 3-33 | 10100 | 3.6 | 44.1 | 1000 |
| 62 | 1-11 | 3-45 | 10100 | 3.9 | 40.7 | 1000 |
| 63 | 1-11 | 3-57 | 10100 | 3.8 | 41.8 | 950 |
| 64 | 1-11 | 4-3 | 10200 | 4.0 | 40.1 | 1000 |
| 65 | 1-11 | 4-4 | 10200 | 4.0 | 40.1 | 1000 |
| 66 | 1-11 | 4-67 | 10200 | 4.0 | 40.1 | 1200 |
| 67 | 1-11 | 4-69 | 10200 | 4.0 | 40.1 | 1100 |
| 68 | 1-20 | 2-2 | 10300 | 4.0 | 40.4 | 900 |
| 69 | 1-20 | 2-3 | 10300 | 3.9 | 41.5 | 900 |
| 70 | 1-20 | 2-33b | 10300 | 3.9 | 41.5 | 1000 |
| 71 | 1-20 | 2-37 | 10300 | 3.9 | 41.5 | 950 |
| 72 | 1-20 | 3-21 | 10100 | 3.9 | 40.7 | 950 |
| 73 | 1-20 | 3-33 | 10100 | 3.9 | 40.7 | 950 |
| 74 | 1-20 | 3-45 | 10100 | 4.0 | 39.7 | 950 |
| 75 | 1-20 | 3-57 | 10100 | 3.9 | 40.7 | 950 |
| 76 | 1-20 | 4-3 | 10200 | 4.0 | 40.1 | 1000 |
| 77 | 1-20 | 4-4 | 10200 | 4.0 | 40.1 | 1000 |
| 78 | 1-20 | 4-67 | 10200 | 4.0 | 40.1 | 1000 |
| 79 | 1-20 | 4-69 | 10200 | 4.0 | 40.1 | 1000 |

**[Table 4]**

| Ex. | First host compd. | Second host compd. | Luminance (cd/m²) | Voltage (V) | Power efficiency (lm/W) | LT70 (h) |
|---|---|---|---|---|---|---|
| 80 | 1-24 | 2-2 | 10300 | 4.0 | 40.4 | 1000 |
| 81 | 1-24 | 2-3 | 10300 | 3.9 | 41.5 | 1000 |
| 82 | 1-24 | 2-33b | 10300 | 3.9 | 41.5 | 1100 |
| 83 | 1-24 | 2-37 | 10300 | 3.9 | 41.5 | 1000 |
| 84 | 1-24 | 3-21 | 10100 | 3.9 | 40.7 | 1000 |
| 85 | 1-24 | 3-33 | 10100 | 3.9 | 40.7 | 1000 |
| 86 | 1-24 | 3-45 | 10100 | 4.0 | 39.7 | 1000 |
| 87 | 1-24 | 3-57 | 10100 | 3.9 | 40.7 | 950 |
| 88 | 1-24 | 4-3 | 10200 | 4.0 | 40.1 | 1100 |
| 89 | 1-24 | 4-4 | 10200 | 4.0 | 40.1 | 1100 |
| 90 | 1-24 | 4-67 | 10200 | 4.0 | 40.1 | 1200 |
| 91 | 1-24 | 4-69 | 10200 | 4.0 | 40.1 | 1100 |
| 92 | 1-27 | 2-2 | 10300 | 3.9 | 41.5 | 1000 |
| 93 | 1-27 | 2-3 | 10300 | 3.9 | 41.5 | 1000 |
| 94 | 1-27 | 2-33b | 10300 | 3.9 | 41.5 | 1100 |
| 95 | 1-27 | 2-37 | 10300 | 3.9 | 41.5 | 1000 |
| 96 | 1-27 | 3-21 | 10100 | 3.8 | 41.8 | 1000 |
| 97 | 1-27 | 3-33 | 10100 | 4.0 | 39.7 | 1000 |
| 98 | 1-27 | 3-45 | 10100 | 3.9 | 40.7 | 1000 |
| 99 | 1-27 | 3-57 | 10100 | 3.9 | 40.7 | 950 |
| 100 | 1-27 | 4-3 | 10200 | 3.9 | 41.1 | 1100 |
| 101 | 1-27 | 4-4 | 10200 | 3.9 | 41.1 | 1100 |
| 102 | 1-27 | 4-67 | 10200 | 3.9 | 41.1 | 1200 |
| 103 | 1-27 | 4-69 | 10200 | 3.9 | 41.1 | 1100 |

**[Table 5]**

| Ex. | First host compd. | Second host compd. | Luminance (cd/m²) | Voltage (V) | Power efficiency (lm/W) | LT70 (h) |
|---|---|---|---|---|---|---|
| 104 | 1-35 | 2-2 | 10300 | 3.9 | 41.5 | 1100 |
| 105 | 1-35 | 2-3 | 10300 | 4.0 | 40.4 | 1200 |
| 106 | 1-35 | 2-33b | 10300 | 4.0 | 40.4 | 1200 |
| 107 | 1-35 | 2-37 | 10300 | 4.0 | 40.4 | 1000 |
| 108 | 1-35 | 3-21 | 10100 | 4.0 | 39.7 | 1000 |
| 109 | 1-35 | 3-33 | 10100 | 4.0 | 39.7 | 1100 |
| 110 | 1-35 | 3-45 | 10100 | 3.9 | 40.7 | 1000 |
| 111 | 1-35 | 3-57 | 10100 | 4.0 | 39.7 | 950 |
| 112 | 1-35 | 4-3 | 10200 | 4.0 | 40.1 | 1000 |
| 113 | 1-35 | 4-4 | 10200 | 4.0 | 40.1 | 1000 |
| 114 | 1-35 | 4-67 | 10200 | 4.0 | 40.1 | 1200 |
| 115 | 1-35 | 4-69 | 10200 | 4.0 | 40.1 | 1100 |
| 116 | 1-37 | 2-2 | 10200 | 4.0 | 40.1 | 1100 |
| 117 | 1-37 | 2-3 | 10200 | 4.0 | 40.1 | 1200 |
| 118 | 1-37 | 2-33b | 10300 | 4.0 | 40.4 | 1200 |
| 119 | 1-37 | 2-37 | 10300 | 3.9 | 41.5 | 1000 |
| 120 | 1-37 | 3-21 | 10100 | 4.0 | 39.7 | 1000 |
| 121 | 1-37 | 3-33 | 10100 | 4.0 | 39.7 | 1100 |
| 122 | 1-37 | 3-45 | 10100 | 3.9 | 40.7 | 1000 |
| 123 | 1-37 | 3-57 | 10100 | 3.8 | 41.8 | 950 |
| 124 | 1-37 | 4-3 | 10200 | 3.9 | 41.1 | 1000 |
| 125 | 1-37 | 4-4 | 10200 | 3.9 | 41.1 | 1000 |
| 126 | 1-37 | 4-67 | 10200 | 3.9 | 41.1 | 1200 |
| 127 | 1-37 | 4-69 | 10200 | 3.9 | 41.1 | 1100 |

**[Table 6]**

| Ex. | First host compd. | Second host compd. | Luminance (cd/m²) | Voltage (V) | Power efficiency (lm/W) | LT70 (h) |
|---|---|---|---|---|---|---|
| 128 | 1-52 | 2-2 | 10200 | 4.0 | 40.1 | 1000 |
| 129 | 1-52 | 2-3 | 10200 | 4.0 | 40.1 | 1000 |
| 130 | 1-52 | 2-33b | 10300 | 4.0 | 40.4 | 1000 |
| 131 | 1-52 | 2-37 | 10300 | 4.0 | 40.4 | 1000 |
| 132 | 1-52 | 3-21 | 10100 | 4.0 | 39.7 | 1000 |
| 133 | 1-52 | 3-33 | 10100 | 4.0 | 39.7 | 1100 |
| 134 | 1-52 | 3-45 | 10100 | 4.0 | 39.7 | 1000 |
| 135 | 1-52 | 3-57 | 10100 | 4.0 | 39.7 | 950 |
| 136 | 1-52 | 4-3 | 10200 | 4.0 | 40.1 | 1100 |
| 137 | 1-52 | 4-4 | 10200 | 4.0 | 40.1 | 1100 |
| 138 | 1-52 | 4-67 | 10200 | 4.0 | 40.1 | 1200 |
| 139 | 1-52 | 4-69 | 10200 | 4.0 | 40.1 | 1000 |
| 140 | 1-63 | 2-2 | 10200 | 4.0 | 40.1 | 1000 |
| 141 | 1-63 | 2-3 | 10200 | 4.0 | 40.1 | 1000 |
| 142 | 1-63 | 2-33b | 10300 | 4.0 | 40.4 | 1000 |
| 143 | 1-63 | 2-37 | 10300 | 4.0 | 40.4 | 1000 |
| 144 | 1-63 | 3-21 | 10100 | 4.0 | 39.7 | 1000 |
| 145 | 1-63 | 3-33 | 10100 | 4.0 | 39.7 | 1100 |
| 146 | 1-63 | 3-45 | 10100 | 4.0 | 39.7 | 1000 |
| 147 | 1-63 | 3-57 | 10100 | 3.9 | 40.7 | 950 |
| 148 | 1-63 | 4-3 | 10200 | 4.0 | 40.1 | 1100 |
| 149 | 1-63 | 4-4 | 10200 | 4.0 | 40.1 | 1100 |
| 150 | 1-63 | 4-67 | 10200 | 4.0 | 40.1 | 1200 |
| 151 | 1-63 | 4-69 | 10200 | 4.0 | 40.1 | 1000 |

**[Table 7]**

| Ex. | First host compd. | Second host compd. | Luminance (cd/m²) | Voltage (V) | Power efficiency (lm/W) | LT70 (h) |
|---|---|---|---|---|---|---|
| 152 | 1-68 | 2-2 | 10200 | 4.0 | 40.1 | 950 |
| 153 | 1-68 | 2-3 | 10200 | 4.0 | 40.1 | 950 |
| 154 | 1-68 | 2-33b | 10300 | 4.0 | 40.4 | 950 |
| 155 | 1-68 | 2-37 | 10300 | 4.0 | 40.4 | 950 |
| 156 | 1-68 | 3-21 | 10100 | 4.0 | 39.7 | 950 |
| 157 | 1-68 | 3-33 | 10100 | 4.0 | 39.7 | 1000 |
| 158 | 1-68 | 3-45 | 10100 | 4.0 | 39.7 | 1000 |
| 159 | 1-68 | 3-57 | 10100 | 4.0 | 39.7 | 950 |
| 160 | 1-68 | 4-3 | 10200 | 4.0 | 40.1 | 1000 |
| 161 | 1-68 | 4-4 | 10200 | 4.0 | 40.1 | 1000 |
| 162 | 1-68 | 4-67 | 10200 | 4.0 | 40.1 | 1100 |
| 163 | 1-68 | 4-69 | 10200 | 4.0 | 40.1 | 950 |
| 164 | 1-76 | 2-2 | 10200 | 4.0 | 40.1 | 950 |
| 165 | 1-76 | 2-3 | 10200 | 4.0 | 40.1 | 950 |
| 166 | 1-76 | 2-33b | 10300 | 4.0 | 40.4 | 950 |
| 167 | 1-76 | 2-37 | 10300 | 4.0 | 40.4 | 950 |
| 168 | 1-76 | 3-21 | 10100 | 3.9 | 40.7 | 950 |
| 169 | 1-76 | 3-33 | 10100 | 4.0 | 39.7 | 1000 |
| 170 | 1-76 | 3-45 | 10100 | 4.0 | 39.7 | 1000 |
| 171 | 1-76 | 3-57 | 10100 | 4.0 | 39.7 | 950 |
| 172 | 1-76 | 4-3 | 10200 | 4.0 | 40.1 | 1000 |
| 173 | 1-76 | 4-4 | 10200 | 4.0 | 40.1 | 1000 |
| 174 | 1-76 | 4-67 | 10200 | 4.0 | 40.1 | 1100 |
| 175 | 1-76 | 4-69 | 10200 | 3.9 | 41.1 | 950 |

**[Table 8]**

| Ex. | First host compd. | Second host compd. | Luminance (cd/m²) | Voltage (V) | Power efficiency (lm/W) | LT70 (h) |
|---|---|---|---|---|---|---|
| 176 | 1-4 | 2-2 | 10300 | 3.9 | 41.5 | 1100 |
| 177 | 1-4 | 2-5 | 10200 | 3.9 | 41.1 | 900 |
| 178 | 1-4 | 2-37 | 10400 | 4.0 | 40.8 | 950 |
| 179 | 1-4 | 3-21 | 10000 | 3.8 | 41.3 | 1000 |
| 180 | 1-4 | 3-41 | 10000 | 3.9 | 40.3 | 950 |
| 181 | 1-4 | 3-47 | 10000 | 3.8 | 41.3 | 1050 |
| 182 | 1-4 | 4-3 | 10200 | 4.0 | 40.1 | 1000 |
| 183 | 1-4 | 4-4 | 10200 | 4.0 | 40.1 | 1000 |
| 184 | 1-4 | 4-67 | 10300 | 4.1 | 39.5 | 1200 |
| 185 | 1-24 | 2-2 | 10300 | 4.0 | 40.4 | 1000 |
| 186 | 1-24 | 2-5 | 10300 | 4.0 | 40.4 | 1000 |
| 187 | 1-24 | 2-37 | 10300 | 3.9 | 41.5 | 1000 |
| 188 | 1-24 | 3-21 | 10100 | 3.8 | 41.8 | 1000 |
| 189 | 1-24 | 3-41 | 10100 | 3.8 | 41.8 | 1000 |
| 190 | 1-24 | 3-47 | 10100 | 3.8 | 41.8 | 1000 |
| 191 | 1-24 | 4-3 | 10200 | 4.0 | 40.1 | 1100 |
| 192 | 1-24 | 4-4 | 10200 | 4.0 | 40.1 | 1100 |
| 193 | 1-24 | 4-67 | 10200 | 4.0 | 40.1 | 1200 |
| 194 | 1-4 | 2-2 | 10300 | 4.0 | 40.4 | 1200 |
| 195 | 1-4 | 2-5 | 10200 | 4.0 | 40.1 | 1000 |
| 196 | 1-4 | 2-37 | 10500 | 4.1 | 40.2 | 1050 |
| 197 | 1-4 | 3-21 | 10000 | 3.8 | 41.3 | 1100 |
| 198 | 1-4 | 3-41 | 10200 | 4.0 | 40.1 | 1050 |
| 199 | 1-4 | 3-47 | 10000 | 3.9 | 40.3 | 1200 |
| 200 | 1-4 | 4-3 | 10500 | 4.1 | 40.2 | 1100 |
| 201 | 1-4 | 4-4 | 10500 | 4.1 | 40.2 | 1100 |
| 202 | 1-4 | 4-67 | 10700 | 4.2 | 40.0 | 1300 |

### Comparative Example 1

An organic EL device was produced in the same manner as in Example 1 except that a compound 2-2 was used alone as a host. The thickness of a light emitting layer and the concentration of a luminescent dopant are the same as those in Example 1.

### Comparative Examples 2 to 14

Organic EL devices were produced in the same manner as in Comparative Example 1 except that each compound shown in Table 9 was used alone as a host.

### Comparative Example 15

An Organic EL device was produced in the same manner as in Example 1 except that a compound A was used as a first host and a compound 2-2 was used as a second host.

### Comparative Examples 16 to 17

Organic EL devices were produced in the same manner as in Comparative Example 15 except that a compound 3-21 or a compound 4-3 was used as a second host.

### Comparative Example 18

An Organic EL device was produced in the same manner as in Example 7 except that a compound B was used as a first host and a compound 2-2 was used as a second host.

### Comparative Examples 19 to 20

Organic EL devices were produced in the same manner as in Comparative Example 18 except that a compound 3-21 or a compound 4-3 was used as a second host.

### Comparative Example 21

An Organic EL device was produced in the same manner as in Example 7 except that a compound C was used as a first host and a compound 2-2 was used as a second host.

### Comparative Examples 22 and 23

Organic EL devices were produced in the same manner as in Comparative Example 21 except that a compound 3-21 or a compound 4-3 was used as a second host.

Evaluation results of the produced organic EL devices are shown in Table 9.

**[Table 9]**

| Comp. Ex. | First host compd. | Second host compd. | Luminance (cd/m²) | Voltage (V) | Power efficiency (lm/W) | LT70 (h) |
|---|---|---|---|---|---|---|
| 1 | - | 2-2 | 9500 | 4.7 | 31.8 | 500 |
| 2 | - | 2-3 | 9000 | 4.7 | 30.1 | 550 |
| 3 | - | 3-21 | 8000 | 4.4 | 28.6 | 500 |
| 4 | - | 3-33 | 8000 | 4.4 | 28.6 | 500 |
| 5 | - | 3-45 | 8000 | 4.4 | 28.6 | 450 |
| 6 | - | 3-57 | 8000 | 4.4 | 28.6 | 450 |
| 7 | - | 4-3 | 10000 | 4.9 | 32.1 | 550 |
| 8 | - | 4-4 | 10000 | 4.9 | 32.1 | 550 |
| 9 | - | 4-67 | 10000 | 4.9 | 32.1 | 600 |
| 10 | A | - | 7000 | 3.0 | 37.2 | 560 |
| 11 | B | - | 7000 | 3.8 | 28.9 | 600 |
| 12 | C | - | 7000 | 3.6 | 30.5 | 600 |
| 13 | D | - | 7000 | 3.2 | 34.4 | 570 |
| 14 | E | - | 6900 | 3.2 | 33.9 | 530 |
| 15 | A | 2-2 | 10000 | 4.3 | 36.5 | 700 |
| 16 | A | 3-21 | 9500 | 4.1 | 36.4 | 700 |
| 17 | A | 4-3 | 10000 | 4.3 | 36.5 | 700 |
| 18 | B | 2-2 | 10500 | 4.6 | 35.9 | 750 |
| 19 | B | 3-21 | 10500 | 4.5 | 36.7 | 700 |
| 20 | B | 4-3 | 10500 | 4.7 | 35.1 | 750 |
| 21 | C | 2-2 | 10000 | 4.5 | 34.9 | 750 |
| 22 | C | 3-21 | 10000 | 4.4 | 35.7 | 700 |
| 23 | C | 4-3 | 10000 | 4.6 | 34.1 | 750 |

It can be seen from Tables 1 to 9 that Examples 1 to 202 have improved power efficiency and lifespan characteristics and show favorable characteristics.

### Example 203

Each thin film was laminated on a glass substrate, on which an anode made of ITO and having a film thickness of 110 nm was formed, with a vacuum degree of 4.0 × 10⁻⁵ Pa through a vacuum vapor deposition method. First, 25 nm thick HAT-CN was formed on the ITO as a hole injection layer, and then 45 nm thick NPD was formed thereon as a hole transport layer. Next, 10 nm thick HT-1 was formed thereon as an electron-blocking layer. Next, a compound 1-1 as a first host and Ir(piq)₂acac as a luminescent dopant were subjected to co-vapor deposition from different vapor deposition sources to form a light emitting layer having a thickness of 40 nm. At this time, the co-vapor deposition was performed under the vapor deposition condition where the concentration of Ir(piq)₂acac was 6.0 wt%. Next, 37.5 nm thick ET-1 was formed thereon as an electron transport layer. Then, 1 nm thick LiF was formed on the electron transport layer as an electron injection layer. Finally, 70 nm thick Al was formed on the electron injection layer as a cathode to produce an organic EL device.

### Examples 204 to 208

Organic EL devices were produced in the same manner as in Example 203 except that each compound shown in Table 10 was used as a host.

### Example 209

Each thin film was laminated on a glass substrate, on which an anode made of ITO and having a film thickness of 110 nm was formed, with a vacuum degree of 4.0 × 10⁻⁵ Pa through a vacuum vapor deposition method. First, 25 nm thick HAT-CN was formed on the ITO as a hole injection layer, and then 45 nm thick NPD was formed thereon as a hole transport layer. Next, 10 nm thick HT-1 was formed thereon as an electron-blocking layer. Next, a compound 1-4 as a first host, a compound 2-2 as a second host, and Ir(piq)₂acac as a luminescent dopant were subjected to co-vapor deposition from different vapor deposition sources to form a light emitting layer having a thickness of 40 nm. At this time, the co-vapor deposition was performed under the vapor deposition conditions where the concentration of Ir(piq)₂acac was 6.0 wt% and the weight ratio of the first host to the second host was 30:70. Next, 37.5 nm thick ET-1 was formed thereon as an electron transport layer. Then, 1 nm thick LiF was formed on the electron transport layer as an electron injection layer. Finally, 70 nm thick Al was formed on the electron injection layer as a cathode to produce an organic EL device.

### Examples 210 to 226

Organic EL devices were produced in the same manner as in Example 209 except that each compound shown in Table 10 was used as a first host and a second host.

Evaluation results of the produced organic EL devices are shown in Table 10. Here, LT95 is the time required for the initial luminance to attenuate to 95% and represents lifespan characteristics.

**[Table 10]**

| Ex. | First host compd. | Second host compd. | Luminance (cd/m²) | Voltage (V) | Power efficiency (lm/W) | LT95 (h) |
|---|---|---|---|---|---|---|
| 203 | 1-1 | - | 3000 | 3.4 | 13.9 | 150 |
| 204 | 1-3 | - | 3000 | 3.4 | 13.9 | 150 |
| 205 | 1-4 | - | 3000 | 3.3 | 14.3 | 180 |
| 206 | 1-11 | - | 3500 | 3.3 | 16.7 | 180 |
| 207 | 1-20 | - | 3000 | 3.4 | 13.9 | 150 |
| 208 | 1-24 | - | 3200 | 3.6 | 14.0 | 180 |
| 209 | 1-4 | 2-2 | 4500 | 3.9 | 18.1 | 250 |
| 210 | 1-4 | 2-5 | 4600 | 3.8 | 19.0 | 250 |
| 211 | 1-4 | 2-37 | 4600 | 3.8 | 19.0 | 200 |
| 212 | 1-4 | 3-21 | 4500 | 3.7 | 19.1 | 250 |
| 213 | 1-4 | 3-41 | 4500 | 3.7 | 19.1 | 250 |
| 214 | 1-4 | 3-47 | 4500 | 3.6 | 19.6 | 200 |
| 215 | 1-4 | 4-3 | 4500 | 3.9 | 18.1 | 220 |
| 216 | 1-4 | 4-4 | 4600 | 3.8 | 19.0 | 220 |
| 217 | 1-4 | 4-67 | 4500 | 3.7 | 19.1 | 200 |
| 218 | 1-24 | 2-2 | 4500 | 3.9 | 18.1 | 200 |
| 219 | 1-24 | 2-5 | 4600 | 3.8 | 19.0 | 250 |
| 220 | 1-24 | 2-37 | 4600 | 3.8 | 19.0 | 200 |
| 221 | 1-24 | 3-21 | 4500 | 3.7 | 19.1 | 200 |
| 222 | 1-24 | 3-41 | 4500 | 3.7 | 19.1 | 250 |
| 223 | 1-24 | 3-47 | 4500 | 3.6 | 19.6 | 200 |
| 224 | 1-24 | 4-3 | 4500 | 3.9 | 18.1 | 220 |
| 225 | 1-24 | 4-4 | 4600 | 3.8 | 19.0 | 250 |
| 226 | 1-24 | 4-67 | 4500 | 3.7 | 19.1 | 200 |

### Comparative Example 24

An organic EL device was produced in the same manner as in Example 203 except that a compound 2-2 was used as a host. The thickness of a light emitting layer and the concentration of a luminescent dopant are the same as those in Example 203.

### Comparative Examples 25 and 37

Organic EL devices were produced in the same manner as in Comparative Example 24 except that each compound shown in Table 11 was used as a host.

### Comparative Examples 38 to 40

Organic EL devices were produced in the same manner as in Example 209 except that a compound A was used as a first host and a compound 2-2, a compound 3-21, or a compound 4-3 was used as a second host.

### Comparative Examples 41 and 43

Organic EL devices were produced in the same manner as in Comparative Examples 38 to 40 except that a compound B was used as a first host.

### Comparative Examples 44 and 46

Organic EL devices were produced in the same manner as in Comparative Examples 38 to 40 except that a compound C was used as a first host.

Evaluation results of the produced organic EL devices are shown in Table 11.

**[Table 11]**

| Comp. Ex | First host compd | Second host compd | Luminance (cd/m²) | Voltage (V) | Power efficiency (lm/W) | LT95 (h) |
|---|---|---|---|---|---|---|
| 24 | - | 2-2 | 3000 | 4.6 | 10.2 | 80 |
| 25 | - | 2-3 | 3500 | 4.5 | 12.2 | 90 |
| 26 | - | 3-21 | 3000 | 4.0 | 11.8 | 100 |
| 27 | - | 3-33 | 3000 | 4.0 | 13.7 | 100 |
| 28 | - | 3-45 | 3000 | 4.1 | 11.5 | 90 |
| 29 | - | 3-57 | 3000 | 4.3 | 11.0 | 90 |
| 30 | - | 4-3 | 3500 | 4.3 | 12.8 | 100 |
| 31 | - | 4-4 | 3500 | 4.4 | 12.5 | 100 |
| 32 | - | 4-67 | 3500 | 4.4 | 12.5 | 100 |
| 33 | A | - | 3000 | 3.4 | 13.9 | 110 |
| 34 | B | - | 3200 | 4.0 | 12.6 | 100 |
| 35 | C | - | 3200 | 4.0 | 12.6 | 100 |
| 36 | D | - | 3000 | 3.8 | 12.4 | 120 |
| 37 | E | - | 3200 | 3.6 | 14.0 | 120 |
| 38 | A | 2-2 | 4000 | 4.4 | 14.3 | 150 |
| 39 | A | 3-21 | 4000 | 3.8 | 14.5 | 150 |
| 40 | A | 4-3 | 4000 | 4.4 | 14.3 | 160 |
| 41 | B | 2-2 | 4200 | 4.5 | 14.7 | 160 |
| 42 | B | 3-21 | 4000 | 4.0 | 15.7 | 160 |
| 43 | B | 4-3 | 4200 | 4.5 | 14.7 | 160 |
| 44 | C | 2-2 | 4000 | 4.5 | 14.0 | 140 |
| 45 | C | 3-21 | 4000 | 4.0 | 15.7 | 140 |
| 46 | C | 4-3 | 4000 | 4.5 | 14.0 | 140 |

It can be seen from Tables 10 and 11 that Examples 203 to 226 have improved power efficiency and lifespan characteristics and show favorable characteristics.

The compounds used in the examples are shown below.

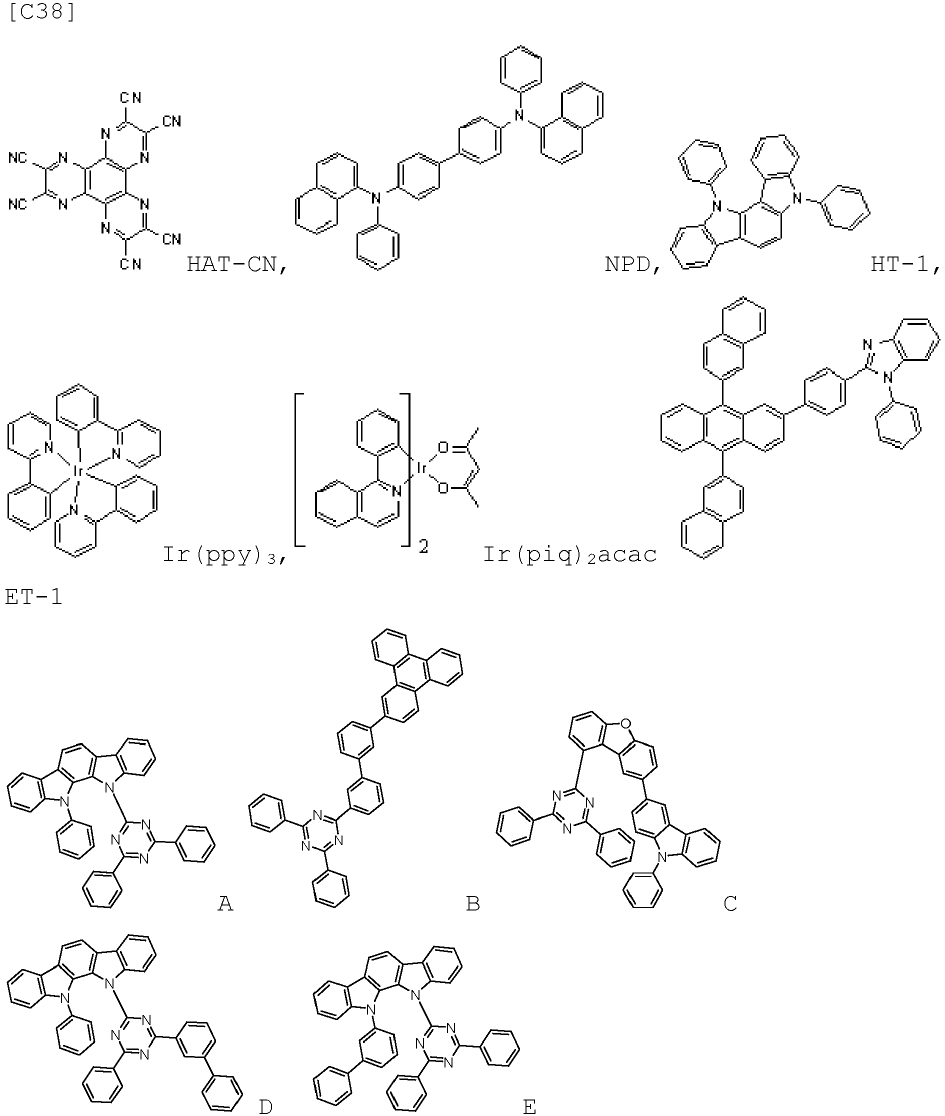

### [Reference Signs List]

- 1: Substrate
- 2: Anode
- 3: Hole injection layer
- 4: Hole transport layer
- 5: Light emitting layer
- 6: Electron transport layer
- 7: Cathode

## Claims

1. A material for an organic electroluminescent device comprising:
an indolocarbazole compound represented by General Formula (1), 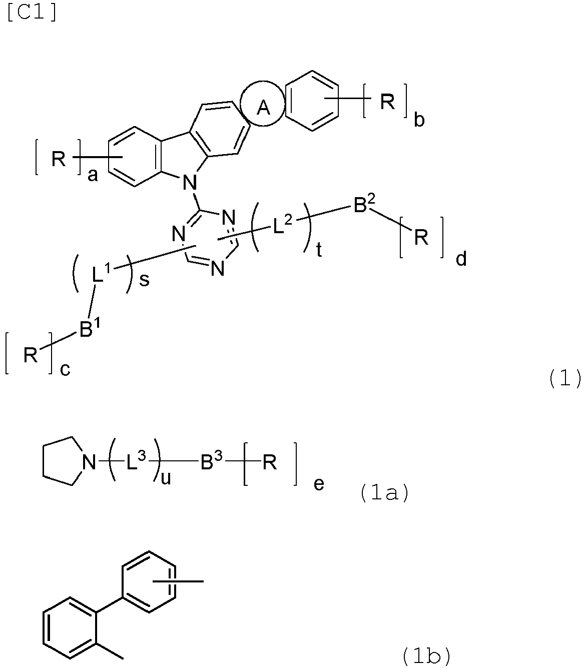
wherein, a ring A is a heterocyclic ring represented by Formula (1a) and condensed with an adjacent ring at an arbitrary position, R's are independently hydrogen, an aliphatic hydrocarbon group having 1 to 10 carbon atoms,
an aromatic hydrocarbon group having 6 to 10 carbon atoms, or an aromatic heterocyclic group having 3 to 12 carbon atoms, L¹, L² and L³ are independently a direct bond, an aromatic hydrocarbon group having 6 to 10 carbon atoms, or
an aromatic heterocyclic group having 3 to 12 carbon atoms, B¹, B² and B³ independently represent a direct bond or a biphenyldiyl group represented by Formula (1b), at least one of B¹ to B³ is a biphenyldiyl group represented by Formula (1b), a, b, c, d, and e each independently represent an integer of 0 to 3, and s, t, and u each independently represent an integer of 1 and 2.

2. The material for an organic electroluminescent device according to claim 1,
wherein, in General Formula (1), B³ is a biphenyldiyl group represented by Formula (1b).

3. The material for an organic electroluminescent device according to claim 1 or 2,
wherein, in General Formula (1), a, b, and c are 0.

4. An organic electroluminescent device comprising an anode, an organic layer, and a cathode are laminated on a substrate,
wherein at least one layer in the organic layer is an organic layer containing the material for an organic electroluminescent device including an indolocarbazole compound represented by General Formula (1) according to any one of claims 1 to 3, and a light emitting layer containing a luminescent dopant material.

5. The organic electroluminescent device according to claim 4,
wherein the organic layer containing the material for an organic electroluminescent device is at least one layer selected from the group consisting of the light emitting layer, an electron transport layer, and a hole-blocking layer.

6. The organic electroluminescent device according to claim 4,
wherein the organic layer containing the material for an organic electroluminescent device is the light emitting layer composed of a vapor deposition layer containing a first host, a second host, and the luminescent dopant material,
wherein the first host is selected from the compounds represented by General Formula (1), and
the second host is selected from compounds represented by General Formula (2), General Formula (3), and General Formula (4) below, 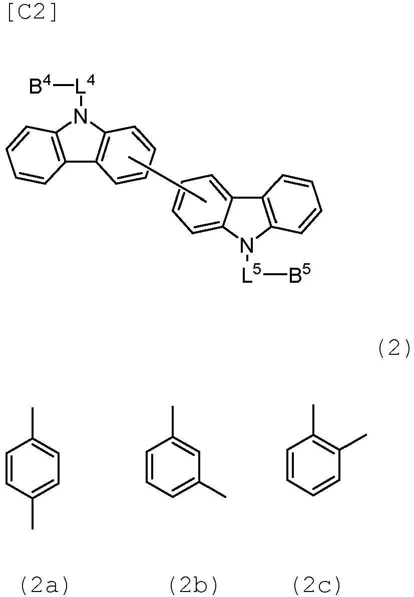
wherein, B⁴ and B⁵ independently represent hydrogen, an aromatic hydrocarbon group having 6 to 14 carbon atoms, or a group in which two aromatic hydrocarbon groups which may be the same as or different from each other are linked, and L⁴ and L⁵ independently represent a phenylene group represented by any of Formulae (2a) to (2c), 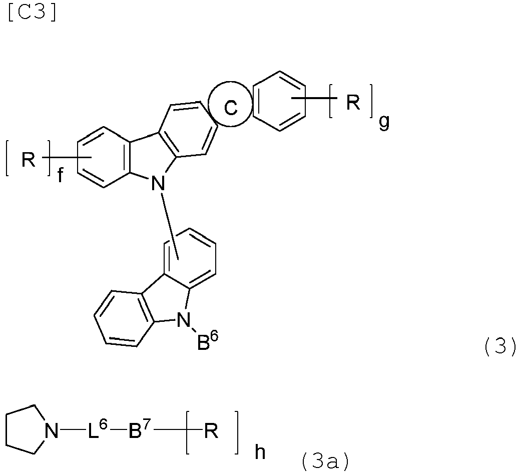
wherein, a ring C is a heterocyclic ring represented by Formula (3a) and condensed with an adjacent ring at an arbitrary position, R's are independently hydrogen, an aliphatic hydrocarbon group having 1 to 10 carbon atoms, an aromatic hydrocarbon group having 6 to 10 carbon atoms, or an aromatic heterocyclic group having 3 to 12 carbon atoms, L⁶ is a direct bond, an aromatic hydrocarbon group having 6 to 10 carbon atoms, or an aromatic heterocyclic group having 3 to 12 carbon atoms, B⁶ is hydrogen, an aromatic hydrocarbon group having 6 to 10 carbon atoms, or an aromatic heterocyclic group having 3 to 12 carbon atoms, B⁷ is an aromatic hydrocarbon group having 6 to 10 carbon atoms or an aromatic heterocyclic group having 3 to 12 carbon atoms, f, g, and h each independently represent an integer of 0 to 3, and 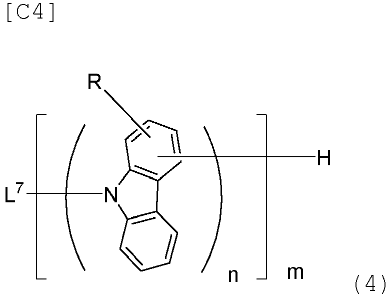
wherein, L⁷ is an m-valent aromatic hydrocarbon group having 6 to 30 carbon atoms, an aromatic heterocyclic group having 3 to 16 carbon atoms, or a linked aromatic group which is obtained by linking 2 to 10 aromatic rings thereof and contains no carbazole ring, R's are independently hydrogen, an alkyl group having 1 to 10 carbon atoms, or a cycloalkyl group having 3 to 11 carbon atoms, m is a substitution number and is an integer of 1 to 3, and n's are numbers of repetitions and each independently an integer of 1 to 4, at least one n is an integer of 2 to 4.

7. The organic electroluminescent device according to claim 6, wherein at least one bonding structure represented by Formula (c1) is included in General Formula (4), 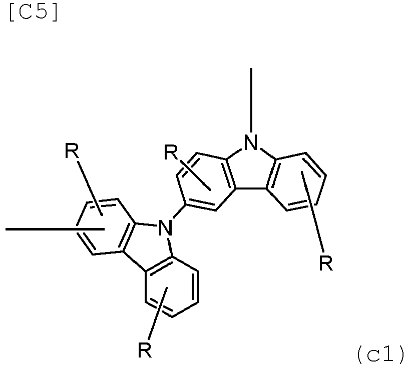 wherein, R has the same meaning as that in General Formula (4) .

8. The organic electroluminescent device according to claim 6 or 7,
wherein the compound represented by General Formula (2) is a compound represented by Formula (5) below, 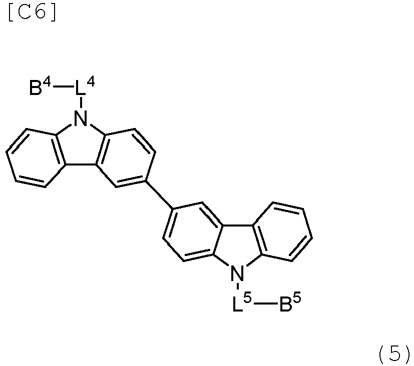 wherein, B⁴, B⁵, L⁴, and L⁵ have the same meaning as those in General Formula (2).

9. The organic electroluminescent device according to any one of claims 6 to 8,
wherein the compound represented by General Formula (3) is a compound represented by Formula (6) or (7) below, 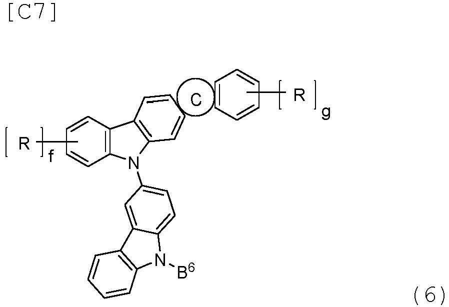 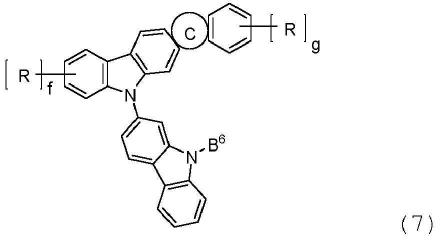 wherein, a ring C, R, B⁶, f, and g have the same meaning as those in General Formula (3).

10. The organic electroluminescent device according to any one of claims 6 to 9,
wherein at least one bonding structure represented by Formula (c2) is included in General Formula (4), 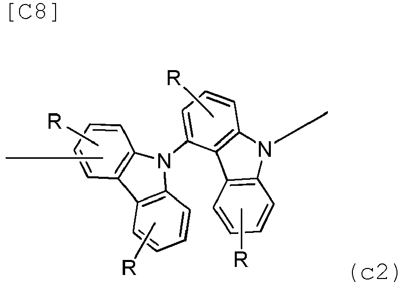 where R has the same meaning as that in General Formula (4).

11. The organic electroluminescent device according to any one of claims 4 to 10, wherein the indolocarbazole compound represented by General Formula (1) is a compound represented by any of Formulae (8) to (11), 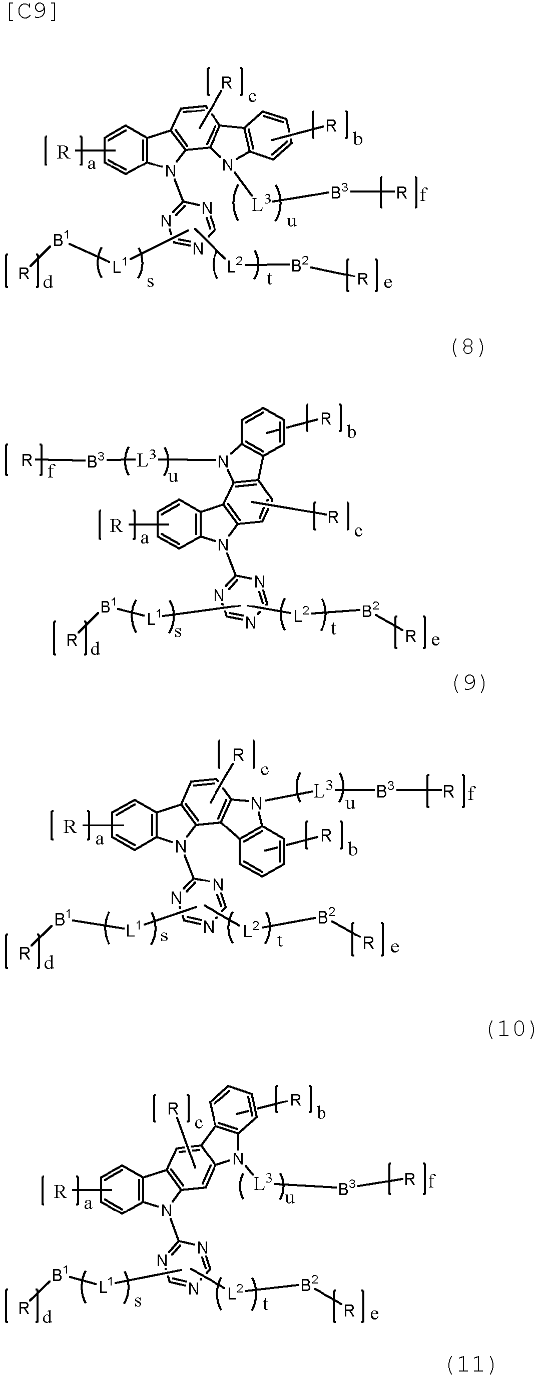 wherein, B¹ to B³, L¹ to L³, R, a to f, and s to u have the same meaning as those in General Formula (1).

12. The organic electroluminescent device according to any one of claims 6 to 11, wherein a proportion of the first host is greater than 20 wt% and less than 55 wt% based on the total amount of the first host and the second host.

13. The organic electroluminescent device according to any one of claims 5 to 12, wherein the luminescent dopant material is an organic metal complex containing at least one metal selected from the group consisting of ruthenium, rhodium, palladium, silver, rhenium, osmium, iridium, platinum, and gold.

14. The organic electroluminescent device according to any one of claims 5 to 12, wherein the luminescent dopant material is a thermally activated delayed fluorescent dopant material.

15. The organic electroluminescent device according to any one of claims 5 to 14, wherein the light emitting layer and the hole-blocking layer adjacent thereto are provided, and the indolocarbazole compound represented by General Formula (1) is contained in the hole-blocking layer.

16. A method for producing an organic electroluminescent device, the method comprising: a step of mixing a first host with a second host to prepare a premixture and then vapor-depositing the host material containing the hosts to form a light emitting layer when producing the organic electroluminescent device according to any one of claims 6 to 15.

17. The method for producing an organic electroluminescent device according to claim 16,wherein a difference in 50% weight reduction temperature between the first host and the second host is within 20°C.
